# EUROPÄISCHE PATENTANMELDUNG

(11) **EP 3 175 866 A1**
(43) Veröffentlichungstag der Anmeldung: **07.06.2017**
(21) Anmeldenummer: 15401120.9
(22) Anmeldetag: 02.12.2015
(51) Int. Cl.: A61L 2/04, A61L 2/07, A61L 2/28, F24D 17/00

(54) **DESINFEKTIONSVORRICHTUNG, VERWENDUNG DESSEN AN ORTSFESTEN LEITUNGEN SOWIE VERFAHREN ZUM DESINFIZIEREN VON ORTSFESTEN LEITUNGEN UND VERFAHREN ZUM AUFBEREITEN DER DESINFEKTIONSVORRICHTUNG**

(71) Anmelder: Werner Besch GmbH, 32120 Hiddenhausen (DE)
(72) Erfinder:
(74) Vertreter: Tanner, Lukas

(57) **Zusammenfassung**

Das Desinfizieren von ortsfesten, insbesondere medizintechnischen Leitungen kann in vielen Fällen nicht auf einfache Weise oder nicht einfach kontrollierbare Weise durchgeführt werden. Offenbart wird eine Desinfektionsvorrichtung zum thermischen Desinfizieren von ortsfesten Leitungen, mit: einem Reservoir für Spülmedium, zum Desinfizieren einer jeweiligen ortsfesten Leitung; wenigstens einer vom Reservoir abgehenden Speiseleitung mit einem Auslass; einer Heizeinrichtung zum Bereitstellen des Spülmediums mit einer bezüglich Raumtemperatur erhöhten Soll-Temperatur; einer mit der Heizeinrichtung gekoppelten Steuerungseinrichtung zum Einstellen, Steuern oder Regeln einer Temperatur des Spülmediums; wenigstens einer an die Steuerungseinrichtung gekoppelten Messeinrichtung zum Erfassen wenigstens einer Messgröße des Spülmediums; einem Rücklauf zum Zurückführen von Spülmedium aus der ortsfesten Leitung zurück in die Desinfektionsvorrichtung mit einem Einlass; wobei die Desinfektionsvorrichtung spülmediumautonom ist. Dies erleichtert die Handhabung und erweitert die Einsatzmöglichkeiten. Ferner wird ein Verfahren zum Desinfizieren von ortsfesten Leitungen oder von der Desinfektionsvorrichtung selbst offenbart.

## Beschreibung

### TECHNISCHES GEBIET

Das technische Gebiet betrifft Vorrichtungen und Verfahren zum Desinfizieren von ortsfesten Leitungen, beispielsweise mit Wasser gefüllte Versorgungsleitungen, welche zu einem Operationssaal führen. Ferner sind Verfahren zum Desinfizieren der Vorrichtung betroffen. Ein Desinfizieren kann z.B. in Vorbereitung auf medizinische Operationen im Krankenhaus erfolgen, bei welchen Sterilität besonders wichtig ist, insbesondere auch direkt nach einer Operation.

### HINTERGRUND

In Krankenhäusern werden ortsfeste Rohrleitungen oder Schläuche für medizintechnische Medien bzw. Fluide genutzt, an welche hohe Reinheitsanforderungen gestellt werden müssen. Beispielsweise besteht bei Hypothermie-Anwendungen insbesondere in Verbindung mit chirurgischen Eingriffen (z.B. am Herzen) die Tendenz, Hypothermie-Geräte nicht mehr unmittelbar neben dem Patienten in einem Operationssaal aufzustellen, sondern allenfalls in einem davon abgegrenzten Vorraum. Hierdurch wird das Risiko gesenkt, mittels der Hypothermie-Geräte Keime in den Operationssaal einzutragen. In der Kardiologie ist dies besonders wichtig.

Jedoch sind dann Zu- und Ableitungen für medizintechnisches Medium bzw. Fluid vom Vorraum in den Operationssaal und zurück erforderlich. Bei Hypothermie-Anwendungen sind dies mit dem Wärmeträger Wasser gefüllte Versorgungsleitungen, welche eine Wärme- oder Kälteversorgung für z.B. einen Wärmetauscher einer Herz-Lungen-Maschine bereitstellen. Üblich sind wenigstens vier ortsfeste Leitungen, insbesondere zweimal Vorlauf und zweimal Rücklauf, z.B. für eine Herz-Lungen-Maschine und einen Operationstisch. Bisher konnte nicht auf einfache Weise kontrolliert werden, ob bzw. in welchem Grade diese Leitungen keimfrei sind. Diese Unsicherheit wird aktuell immer weniger akzeptabel, insbesondere hinsichtlich Bakterien, deren Einfluss auf den menschlichen Organismus bisher nicht ausreichend bekannt ist. Es besteht häufig die Anforderung, zumindest der Trinkwasserverordnung gerecht zu werden.

Einer der Gründe für die erhöhten Anforderungen an die Reinheit solcher Leitungen ist das Risiko, Mykobakterien in den Operationssaal einzutragen oder sogar an den Patienten heranzuführen. Eine Infektion mit Mykobakterien kann in vielen Fällen nicht auf einfache Weise diagnostiziert werden. Es wird sogar befürchtet, dass sich durch Mykobakterien verursachte Symptome erst nach vielen Monaten oder Jahren zeigen. Diese Situation erschwert eine Diagnose und erhöht die Unsicherheit hinsichtlich der Herkunft der Bakterien. Somit müssen noch höhere Anforderungen an die Reinheit von Zu- und Ableitungen gestellt werden.

In diesem Zusammenhang sind insbesondere Mykobakterien oder ein sich auf den Innenwandungen von medizintechnischen Leitungen ablagernder Biofilm erwähnenswert. Der Biofilm ist auch insofern problematisch, als viele ortsfeste Leitungen kontinuierlich Wasser führen bzw. mit Wasser gefüllt sind. Das Wasser steht über eine lange Dauer in diesen Leitungen. Ein Biofilm sollte also möglichst rückstandslos entfernt werden. Insbesondere sollten auch Mykobakterien möglichst vollständig abgetötet werden.

Es sind diverse Verfahren und Vorrichtungen zum Desinfizieren von medizinischen Instrumenten in dafür vorgesehenen Behältern oder Kammern bekannt, sei es durch Applizieren von flüssigen Desinfektionsmitteln oder von Dampf.

Die Patentschrift DE 3838046 C2 beschreibt ein Verfahren zum thermischen Desinfizieren einer Wasserversorgunganlage und stellt sich die Aufgabe, das Desinfizieren ohne Änderungen am Aufbau der Anlage zu ermöglichen. Ein Warmwasserbereiter kann Wasser auf erhöhter Temperatur bereitstellen, welches in die Versorgungsanlage eingeleitet werden kann.

Bisher nicht zufriedenstellend geklärt ist jedoch, mittels welcher Vorrichtungen oder welchem Verfahren vergleichsweise voluminöse ortsfeste Leitungen desinfiziert werden könnten, und zwar möglichst derart, dass ein Desinfizieren schnell oder ohne besondere Vorkenntnisse oder ohne besondere Infrastruktur auch nach dem Tagesbetrieb in z.B. Krankenhäusern realisierbar ist. In Herzkliniken wäre dies während einer Zeitspanne von üblicherweise sechs bis acht Stunden nachts möglich. Dabei wird auch eine Überprüfbarkeit der Desinfektionsvorgänge gewünscht, insbesondere zwecks Rückverfolgung von Keimquellen.

Aufgabe ist, eine Vorrichtung oder ein Verfahren bereitzustellen, womit das Desinfizieren ortsfester Leitungen auf einfache Weise erfolgen kann, insbesondere ohne Risiko von Infektionen aus der Umgebung, oder ohne technischen Aufwand, oder ohne aufwändige Vor- und Nacharbeiten, oder auch ohne besondere fachliche Vorkenntnisse eines Bedieners der Vorrichtung. Auch ist es Aufgabe, eine Vorrichtung so auszuführen, dass nachvollzogen oder rekonstruiert werden kann, in welcher Art und Weise ein Desinfizieren erfolgt ist. Nicht zuletzt ist es Aufgabe, eine Vorrichtung so auszuführen, dass deren relevante Komponenten keimfrei gehalten werden können, selbst falls die Vorrichtung zumindest zeitweise auch in nicht keimfreien Bereichen angeordnet ist.

### ZUSAMMENFASSUNG

Die Aufgabe wird durch eine Desinfektionsvorrichtung gemäß Anspruch 1 bzw. dessen Verwendung sowie durch ein Verfahren gemäß dem nebengeordneten Verfahrensanspruch gelöst. Vorteilhafte Weiterbildungen werden in den jeweiligen Unteransprüchen erläutert. Die Merkmale der im Folgenden beschriebenen Ausführungsbeispiele sind miteinander kombinierbar, sofern dies nicht explizit verneint ist.

Die Aufgabe wird gelöst durch eine Desinfektionsvorrichtung zum thermischen Desinfizieren von Innenflächen von ortsfesten Leitungen, insbesondere medizintechnischen Leitungen, mit:
einem Reservoir für Spülmedium, insbesondere zusatzfreies Wasser, zum Desinfizieren einer jeweiligen ortsfesten Leitung;
wenigstens einer vom Reservoir abgehenden Speiseleitung mit einem Auslass zum Kuppeln der Desinfektionsvorrichtung an eine jeweilige zu desinfizierende ortsfeste Leitung;
einer Heizeinrichtung zum Bereitstellen des Spülmediums mit einer bezüglich Raumtemperatur erhöhten Soll-Temperatur, insbesondere mindestens 73°C, insbesondere im Reservoir, am Auslass und/oder am Einlass; einer mit der Heizeinrichtung gekoppelten Steuerungseinrichtung zum Einstellen, Steuern und/oder Regeln einer Temperatur des Spülmediums;
wenigstens einer an die Steuerungseinrichtung gekoppelten Messeinrichtung zum Erfassen wenigstens einer Messgröße des Spülmediums, insbesondere einer Temperatur;
einem Rücklauf zum Zurückführen von Spülmedium aus der ortsfesten Leitung zurück in die Desinfektionsvorrichtung mit einem Einlass zum Kuppeln der jeweiligen zu desinfizierenden ortsfesten Leitung an die Desinfektionsvorrichtung bzw. an den Rücklauf; wobei die Desinfektionsvorrichtung spülmediumautonom ist. Insbesondere kann das Reservoir kann derart dimensioniert sein, dass die Desinfektionsvorrichtung spülmediumautonom ist.

Die Desinfektionsvorrichtung ist eingerichtet zum Desinfizieren von Innenoberflächen bzw. von langgestreckten engen, vergleichsweise voluminösen Hohlräumen mit großem Längen-Breiten-Verhältnis. Dabei können mehrere ortsfeste Leitungen in Reihe miteinander verkuppelt werden und eine längere Leitung bilden, z.B. vier medizintechnische Leitungen, die jeweils paarweise mit einem Verbindungsschlauch miteinander verbunden sind. Diese Anordnung erfordert drei Verbindungsschläuche, die z.B. aus Silikon bestehen können.

Im Gegensatz zum Desinfizieren oder Sterilisieren von z.B. Endoskopen oder anderen medizintechnischen Instrumenten in Sterilisierkammern ist die Desinfektionsvorrichtung eingerichtet, Hohlräume außerhalb bzw. extern von der Desinfektionsvorrichtung zu desinfizieren, d.h., nicht in die Desinfektionsvorrichtung integrierte und auch nicht von der Desinfektionsvorrichtung umgebene Hohlräume. Hierzu stellt die Desinfektionsvorrichtung Spülmedium mit einem bestimmten Volumenstrom bereit, insbesondere auch in kontinuierlicher Art und Weise. Ein kontinuierlich anhaltender, beispielsweise konstanter Spülmedium-Volumenstrom kann eine gute Überwachung bzw. Kontrolle des Desinfektionsvorgangs ermöglichen.

Die Desinfektionsvorrichtung kann eingerichtet sein, die Desinfizierung ohne Zuführen von Spülmedium ausschließlich mittels Energiezufuhr durchzuführen, insbesondere mittels über eine Stromleitung zugeführter Energie. Wahlweise kann die Desinfektionsvorrichtung auch einen Energiespeicher aufweisen und auch energieautark sein, zumindest in Bezug auf einen oder mehrere Desinfektionsvorgänge. Dann nämlich kann eine Desinfizierung bzw. Eigen-Desinfektion vollständig unabhängig von irgendwelchen Versorgungsleitungen oder Energieströmen erfolgen, also an einem beliebigen Ort.

Die Autonomie der Desinfektionsvorrichtung ermöglicht das Desinfizieren von zahlreichen ortsfesten Leitungen eines Krankenhauses in kurzer Zeit nacheinander, z.B. jede Nacht die Leitungen zu zwei Operationssälen. Eine zeiteffiziente Desinfektion kann dabei insbesondere bei großen Herzzentren mit z.B. zehn oder mehr Operationssälen Vorteile liefern.

Es hat sich gezeigt, dass die Kontrolle hinsichtlich Bakterien sehr erleichtert werden kann, wenn Desinfektionsvorgänge standardisiert und dokumentiert ablaufen. Die Desinfektionsvorrichtung ist zum thermischen Desinfizieren basierend auf einem vollautomatischen Desinfektionsablauf mit Dokumentation des Desinfektionsortes (nicht manipulierbar), der Desinfektionstemperatur, der Desinfektionszeit, der Umlaufmenge von Spülmedium eingerichtet, wobei wahlweise auch eine vollautomatische Probeentnahme vorgenommen werden kann. Die Desinfektionsvorrichtung ist eingerichtet, den Desinfektionsprozess vollständig ohne Bedienpersonal auszuführen. Nach dem Einsatz und dem Transport der Desinfektionsvorrichtung zu einem Servicepunkt kann dort die vollautomatische Entleerung, Reinigung und Desinfektion der Desinfektionseinrichtung erfolgen (Eigen-Desinfektion). Auch dieser Desinfektionsvorgang kann vollständig dokumentiert werden.

Das Reservoir kann derart dimensioniert sein, dass die Desinfektionsvorrichtung keine Zuführung von Spülmedium von extern erfordert, sondern spülmediumautonom bleibt, selbst wenn eine vergleichsweise voluminöse Leitung desinfiziert werden soll. Das Reservoir kann z.B. aus Edelstahl gefertigt sein. Edelstahl ist hinsichtlich Keimfreiheit vorteilhaft. Das Reservoir kann z.B. temperaturbeständig bis 110°C, 150°C, 200°C oder auch deutlich über 200°C sein. Das Reservoir kann dabei vergleichsweise voluminös ausgebildet sein und z.B. ein Volumen im Bereich von 120 oder 150Litern aufweisen. Das Reservoir kann zur Aufnahme bzw. zum Vorhalten eines Volumens entsprechend einem Vielfachen des Volumens der jeweiligen ortsfesten Leitung sein, beispielsweise dem 3fachen oder 4fachen oder 5fachen (Faktor 3 bis 5). Hierdurch kann ein vergleichsweise großer Energiepuffer bereitgestellt werden. Dies ermöglicht nicht zuletzt auch das Halten der gewünschten Mindest-Temperatur (insbesondere 73°C), insbesondere wenn das Spülmedium im Kreislauf geführt wird. Ein Reservoir mit einem Vielfachen des Volumens der ortsfesten Leitung liefert auch eine große Sicherheit. Insbesondere kann ausgeschlossen werden, dass eingebrachtes und im Kreislauf geführtes Spülmedium den Energieinhalt des Spülmediums im Reservoir so weit verringert, dass die Temperatur unter die Mindest-Temperatur sinkt. Es kann eine Energiemenge bereitgestellt werden, welche mindestens um eine Differenz größer ist als die Energiemenge, die beim Durchströmen der ortsfesten Leitung an die Umgebung abgegeben wird bzw. verloren geht. Es kann ausgeschlossen werden, dass der Desinfektionsvorgang auf höherer Temperatur wiederholt werden muss.

Die Desinfektionsvorrichtung kann auch einen Rücklaufbehälter zur Aufnahme von gebrauchtem Spülmedium bereitstellen. Gebrauchtes Spülmedium kann zunächst in das Reservoir (Kreislaufführung) und/oder zumindest teilweise direkt in den Rücklaufbehälter geführt werden. Der Rücklauf kann zum Rücklaufbehälter und/oder Reservoir führen.

Auch der Rücklaufbehälter kann dabei vergleichsweise voluminös ausgebildet sein und z.B. ein Volumen im Bereich von 125 oder 150L aufweisen. Der Rücklaufbehälter kann derart dimensioniert sein, dass die Desinfektionsvorrichtung spülmediumautonom ist und auch keinen Anschluss zum Ableiten von gebrauchtem Spülmedium erfordert.

Bevorzugt ist der Rücklaufbehälter getrennt bzw. separat vom Reservoir angeordnet. Dies ermöglicht eine Trennung von ungebrauchtem Spülmedium und gebrauchtem Spülmedium. Ungebrauchtes, keimfreies Spülmedium kann wahlweise zu einem beliebigen Verfahrenszeitpunkt bereitgestellt werden. Wahlweise kann ein Teil des im Reservoir bereitgehaltenen Spülmediums im Kreislauf geführt werden, beispielsweise in einem zweiten Schritt, nachdem in einem ersten Schritt ein Desinfizieren einer bakteriell besonders stark belasteten Leitung durch Einleiten von Spülmedium direkt in den Rücklaufbehälter erfolgt. Wahlweise kann das gesamte im Reservoir bereitgehaltene Spülmedium im Kreislauf geführt werden.

In vorteilhafter Weise ist das Reservoir oberhalb vom bzw. über dem Rücklaufbehälter angeordnet. Dies ermöglicht ein Entleeren des Reservoirs in den Rücklaufbehälter ohne Energieeintrag, insbesondere durch Gravitationskräfte. Unten am Rücklaufbehälter kann ein Ablauf bzw. Entwässerungsrohr vorgesehen sein. Die gesamte Desinfektionsvorrichtung kann dann ohne Energieeintrag entleert werden.

Der Rücklaufbehälter bzw. eine damit verbundene Armatur kann temperaturbeständig bis weit über 100°C oder bis über 200°C sein. Eine Temperaturbeständigkeit im Bereich von 150°C oder 200°C ermöglicht auch das Desinfizieren dieser Komponenten mittels Spülmedium in der Gasphase.

Der Auslass kann z.B. an der Speiseleitung oder einem damit verbundenen Schlauch vorgesehen sein. Am Einlass bzw. Auslass kann jeweils eine genormte, beidseitig dicht schließende Steckkupplung vorgesehen sein. Ein Temperatursensor kann z.B. an der Speiseleitung und/oder am Rücklauf angeordnet sein.

Die Steuerungseinrichtung kann eine Recheneinheit (CPU) aufweisen. Die Steuerungseinrichtung kann ein Steuergerät aufweisen. Die Steuerungseinrichtung kann an einen Daten- bzw. Programmspeicher gekuppelt sein oder diesen aufweisen. Im Speicher können Programmdaten und/oder Messwerte oder vorgegebene Soll-Werte abgelegt sein. Beispielsweise sind Programmdaten für mehrere Spülprogramme zum Durchführen automatischer Spülung bzw. automatischer Desinfektion hinterlegt. Wahlweise sind auch Programmdaten für wenigstens ein Aufbereitungsprogramm zum automatischen Aufbereiten der Desinfektionsvorrichtung hinterlegt. Auch können Kalender- oder Zeitdaten oder Zeitprogramme zum autonomen Herstellen der Betriebsbereitschaft der Desinfektionsvorrichtung, insbesondere zur autonomen Eigen-Desinfektion hinterlegt sein.

Als ortsfeste, insbesondere medizintechnische Leitung ist dabei ein Rohr, ein Schlauch oder eine beliebige andere vorinstallierte, ortsfeste Versorgungsleitung für z.B. im Zusammenhang mit Operationen benötigte desinfizierte oder sterile Medien bzw. Fluide zu verstehen, insbesondere in Krankenhäusern. Die ortsfeste Leitung kann auch als Hausleitung oder als Krankenhausleitung bezeichnet werden. Ein Beispiel für solche Leitungen sind Zu- und Ableitungen für Hypothermiegeräte, welche in einem Raum installiert sind, der einem Operationssaal bzw. Operationsraum vorgelagert ist, z.B. in einem Waschraum. Die Zu- und Ableitungen müssen jedoch strengen Reinheitsanforderungen genügen. Die ortsfeste Leitung kann als Wärmeträgerleitung für Hypothermiegeräte ausgeführt sein, mit entsprechenden fluiddichten Kupplungen. Die ortsfeste Leitung kann ein (Innen-)Volumen von z.B. 5L bis 10L aufweisen. Mehrere aneinander gekuppelte Leitungen können auch ein Volumen von z.B. 15L oder 20L aufweisen. Ein weiteres Beispiel für eine solche ortsfeste Leitung ist eine Wasserleitung, welche der Trinkwasserverordnung genügen muss.

An einem (jeweiligen) offenen Ende kann die ortsfeste Leitung eine Kupplung aufweisen, insbesondere eine Edelstahl-Kupplung, welche beidseitig dicht schließend ist. Dies ermöglicht ein Kuppeln ohne Wasseraustritt. Zwei offene Enden einer jeweiligen ortsfesten Leitung können mit dem Auslass und dem Einlass verkuppelt werden. Zwei nebeneinander liegende Leitungen können z.B. mit einem Silikonschlauch verbunden werden, insbesondere auch mit einem in einem Operationssaal angeordneten Schlauch. Dies kann z.B. ein Kardio-Techniker durchführen.

Das Spülmedium kann dann die Leitung vollständig durchströmen. Dies ermöglicht auch, den Desinfektionsvorgang mittels einstellbarer Strömungsgeschwindigkeiten, Druck, oder Verweilzeiten zu steuern und dessen Effektivität oder Effizienz zu optimieren. Als Spülmedium kann z.B. Wasser mit zumindest Trinkwasserqualität verwendet werden.

Als Messeinrichtung ist dabei eine Einrichtung zum Erfassen von einer oder mehreren Messgrößen bzw. Messwerten zu verstehen. Die Messeinrichtung kann einen oder mehrere Sensoren jeweils für eine oder mehrere Messgrößen aufweisen. Die Sensoren können an unterschiedlichen Positionen oder Komponenten der Desinfektionsvorrichtung vorgesehen sein.

Als Speiseleitung ist dabei eine Leitung zum Zuführen oder Weiterleiten von Spülmedium zu verstehen. Die Speiseleitung umfasst zumindest solche Leitungsabschnitte, welche vom Reservoir zum Auslass führen.

Als "spülmediumautonom" ist dabei eine Vorrichtung zu verstehen, welche keine Spülmedium- oder Desinfektionsmittelzuführung benötigt, um den Desinfektionsvorgang durchführen zu können. Während eines oder mehrerer Desinfektionsvorgänge kann das Spülmedium allein durch die Desinfektionsvorrichtung bereitgestellt werden. Die Desinfektionsvorrichtung ist dann, zumindest zeitweise, autark hinsichtlich Spülmedium. Die Desinfektionsvorrichtung ist selbstversorgend, beispielsweise auch bezüglich eines vergleichsweise großen Volumens einer Vielzahl von ortsfesten Leitungen. Hierzu kann das Volumen des Reservoirs ein Vielfaches des Volumens der zu desinfizierenden Leitung(en) aufweisen. Das Reservoir weist z.B. ein Volumen im Bereich von 100L oder 150L oder auch bis zu 250L auf. Mittels einer spülmediumautonomen Desinfektionsvorrichtung können beispielsweise sämtliche ortsfesten Leitungen einer Krankenhaus-Station desinfiziert werden, ohne dass eine Spülmediumversorgung erforderlich wäre. Dies ermöglicht nicht zuletzt, eine Krankenhausinfrastruktur schlanker auszuführen.

Indem mehrere ortsfeste Leitungen mittels einer einzigen Füllung des Reservoirs desinfizierbar sind, kann auch ein großer Teil der ansonsten erforderlichen Reinigungsarbeit im Bereich der Leitungsanschlüsse im jeweiligen Vorraum oder Operationssaal entfallen.

Ein von einer Spülmedium-Versorgung unabhängiges Gerät liefert dabei auch den Vorteil, dass eine Keimfreiheit des Spülmediums leichter überwacht oder protokolliert werden kann. Die Desinfektionsvorrichtung ist auch insofern autark, als sie in desinfiziertem, keimfreiem Zustand bereitgestellt werden kann. Insbesondere ist die Desinfektionsvorrichtung für eine Eigen-Desinfektion eingerichtet. Dies vermindert ein Risiko, beim Desinfizieren Keime oder Bakterien in ortsfeste Leitungen einzutragen. Kupplungen oder Anschlüsse können beim Desinfizieren ebenfalls auf eine Mindesttemperatur gebracht werden. Dies kann z.B. dadurch sichergestellt werden, dass die Desinfektionsvorrichtung sowohl am Auslass als auch am Einlass Temperatursensoren aufweist.

Die Desinfektionsvorrichtung kann als verlagerbares, mobiles Standgerät ausgebildet sein, insbesondere zur Aufnahme von Gewichtskräften deutlich über 100kg oder 200kg. Die Desinfektionsvorrichtung kann fahrbar, insbesondere rollbar sein bzw. Rollen oder Räder zum Verlagern aufweisen. Wenigstens eines der Räder kann blockierbar sein bzw. an eine Bremseinrichtung gekoppelt sein. Jedes Rad kann z.B. für eine statische Belastung von 300-500kg ausgelegt sein, mindestens jedoch 100kg. Die Auslegung der Räder kann entsprechend der zulässigen Bodenbelastung erfolgen, insbesondere um zu hohe Druckspitzen zu vermeiden. Die Räder können elektrisch ableitend sein.

Die Desinfektionsvorrichtung kann einen motorischen Antrieb zum gesteuerten Verlagern aufweisen. Beispielsweise sind einzelne Rollen antreibbar. Die Desinfektionsvorrichtung kann ein beträchtliches Gesamtgewicht aufweisen. Falls Reservoir, Kaltbehälter und Rücklaufbehälter allesamt vollständig gefüllt sind, kann sich ein Gesamtgewicht von z.B. 250-300kg ergeben, oder auch im Bereich von bis zu 500kg. Ein oder mehrere motorische Antriebe, welche wahlweise auch über eine Eingabemaske oder z.B. einen Joystick ansteuerbar sind, können das Verlagern der Desinfektionsvorrichtung erleichtern oder beschleunigen. Ebenso können automatische Bremsen vorgesehen sein, und/oder ein jeweiliger Antrieb kann auch zum Ausüben einer Bremskraft eingerichtet sein.

Die Desinfektionsvorrichtung kann eine Anzeige- und Eingabeeinheit aufweisen, mittels welcher eine Eingabemaske zum Anzeigen von z.B. Messwerten und Eingeben von z.B. Soll-Werten bereitgestellt wird. Beispielsweise kann ein Nutzer eine gewünschte Desinfektionstemperatur vorgeben, z.B. einen Mindestwert von 73°C und/oder eine Mindest-Desinfektionszeit, insbesondere auf der vorgegebenen Desinfektionstemperatur.

Im Folgenden werden Weiterbildungen und Ausführungsbeispiele beschrieben.

Gemäß einem Ausführungsbeispiel ist die Desinfektionsvorrichtung eingerichtet, mittels der Speiseleitung und dem Rücklauf in Verbindung mit einer jeweiligen ortsfesten Leitung einen bezüglich der Desinfektionsvorrichtung auch extern angeordneten desinfizierbaren, hermetischen bzw. abdichtbaren Umlauf zu bilden. Anders ausgedrückt: Die Desinfektionsvorrichtung kann zum Desinfizieren von extern von bzw. außerhalb der Desinfektionsvorrichtung angeordneten Leitungen eingerichtet sein, insbesondere auch zum Dokumentieren von Temperatur-, Druck- und/oder Durchflusswerten bezüglich eines externen Abschnitts des Umlaufs. Der Umlauf kann auch eine Kreislaufführung ermöglichen. Der gesamte Umlauf kann temperaturbeständig bis z.B. 110°C oder auch 150°C oder 200°C sein. Eine Kreislaufführung hat den Vorteil, dass mit einem vergleichsweise geringen Volumen auch eine große Durchflussrate realisierbar ist. Auch kann ein Energiepuffer bereitgestellt werden. Indem ein Umlauf gebildet wird, kann z.B. durch Erfassen von Messwerten am Einlass ermittelt werden, welcher Temperatur und welchem Durchfluss oder Druck ein jeweiliger Leitungsabschnitt ausgesetzt war. Es kann zweckdienlich sein, die Messwerte zumindest am Einlass und am Auslass zu erfassen. Insbesondere die am Einlass erfassten Messwerte können herangezogen werden, um die Art und Weise der Desinfektion zu beurteilen und zu dokumentieren.

Die Desinfektionsvorrichtung kann zum Bereitstellen einer Soll-Temperatur am Auslass und/oder am Einlass eingerichtet sein. Dies ermöglicht, das Desinfizieren außerhalb der Desinfektionsvorrichtung durchzuführen, also nicht in einer Kammer innerhalb der Desinfektionsvorrichtung, sondern in externen Abschnitten des Umlaufs. Das Einhalten einer Soll-Temperatur am Einlass, also im Bereich des Rücklaufs, kann dabei sicherstellen, dass externe Abschnitte des Umlaufs, also die ortsfeste Leitung, einer bestimmten Mindest-Temperatur ausgesetzt waren. Dabei wird bevorzugt auch die Zeit erfasst.

Gemäß einem Ausführungsbeispiel ist die Desinfektionsvorrichtung zum automatischen Spülen und Desinfizieren einer jeweiligen ortsfesten Leitung durch einen Umlauf oder im Kreis (Kreislauf-Führung) eingerichtet, indem der Heizeinrichtung und/oder einer Fördereinrichtung der Desinfektionsvorrichtung von der Steuerungseinrichtung in Abhängigkeit von an der Speiseleitung und am Rücklauf erfassten Messwerten eine oder mehrere Betriebsgrößen vorgebbar sind, insbesondere Leistungsabgabe, Förderleistung, und/oder Betriebsintervall. Dabei kann die Steuerungseinrichtung zum Einstellen, Steuern und/oder Regeln wenigstens einer Messgröße in Kommunikation mit wenigstens einem Sensor der Messeinrichtung stehen.

Gemäß einem Ausführungsbeispiel weist die Desinfektionsvorrichtung eine Fördereinrichtung eingerichtet zum Fördern von Spülmedium durch die Speiseleitung und eine jeweilige ortsfeste Leitung auf. Die Fördereinrichtung kann zum Fördern von Fluid, also sowohl Flüssigkeit als auch Gas eingerichtet sein.

Die Fördereinrichtung kann über eine oder mehrere Leitungen mit dem Spülmedium-Reservoir und dem Kaltbehälter verbunden sein. Die Fördereinrichtung kann wenigstens eine Pumpe aufweisen, insbesondere zum Bereitstellen des Spülmediums mit vorgegebenem Druck am Auslass.

Die Fördereinrichtung kann eingerichtet sein, einen Druck im Bereich von z.B. 3bar aufzubauen. Die Fördereinrichtung kann dazu ausgelegt sein, ortsfeste Leitungen mit einer Länge im Bereich von z.B. 200m zu spülen. Die Fördereinrichtung kann eingerichtet sein, ortsfeste Leitungen zu spülen, die sich in vertikaler Richtung über eine Höhe von z.B. 15m oder mehr erstrecken. Dies ermöglicht z.B., ausgehend von einem Stockwerk auch Leitungen zu spülen, welche sich in andere Stockwerke erstrecken.

Die Desinfektionsvorrichtung kann z.B. für den Nenndruck PN16 ausgelegt sein und für einen Arbeitsdruck von 10bar zugelassen sein. Dann ist das Spülen von Leitungen mit bis zu ca. 100m Höhenunterschied möglich.

Die Fördereinrichtung kann einen Kompressor zum Bereitstellen von Druckluft aufweisen, insbesondere keimfreier, medizintechnischer Druckluft aus dem Versorgungsnetz eines Krankenhauses.

Die Desinfektionsvorrichtung kann einen Druckluftanschluss und damit verbundene Druckluftleitungen aufweisen, insbesondere für Drücke im Bereich von 5bara.

Gemäß einem Ausführungsbeispiel ist das Reservoir ein Heißreservoir zum Bereitstellen und Vorhalten von Spülmedium mit einer Temperatur von mindestens 73°C, insbesondere mindestens 102°C. Das Heißreservoir ist eingerichtet zum Bereitstellen von Spülmedium mit einer Temperatur im Bereich von 73°C bis über 100°C, wobei jeder der dazwischenliegenden Temperaturwerte bereitgestellt oder gehalten werden kann. Eine Temperatur über 100°C kann dazu dienen, Spülmedium in der Gasphase bereitzustellen. Das Heißreservoir kann ein Spülmedium-Volumen entsprechend einem Vielfachen des Volumens der zu desinfizierenden Leitung(en) bereithalten, insbesondere mit einer Temperatur über 73°C. Indem ein gewisses Volumen an Spülmedium, insbesondere Heißwasser vorgehalten werden kann, insbesondere zumindest in Trinkwasserqualität, kann ein spülmediumautonomes Gerät auch für vergleichsweise lange oder voluminöse ortsfeste Leitungen bereitgestellt werden, insbesondere ohne chemische Zusätze.

Das Reservoir kann zur Aufnahme eines vergleichsweise großen Volumens von Spülmedium in der Flüssigphase eingerichtet sein. Das Reservoir kann zur Weiterleitung von Gewichtskräften deutlich über 100kg an einem Rahmen oder Boden der Desinfektionsvorrichtung abgestützt sein.

Als Heißreservoir ist dabei ein Behälter zu verstehen, in welchem heißes Spülmedium vorgehalten werden kann und/oder auf eine gewünschte zum Desinfizieren geeignete Temperatur erwärmt werden kann.

Mittels eines Heißreservoirs ist es auch möglich, ein großes Spülmedium-Volumen mit großem Energie- bzw. Wärmeinhalt in kurzer Zeit durch die jeweilige ortsfeste Leitung zu spülen.

Die Heizeinrichtung kann eingerichtet sein, ein Spülmedium-Volumen von 60L oder 100L oder 150L oder bis zu 250L mit der Wärmekapazität von Wasser auf eine Temperatur von mindestens 70°C, insbesondere mindestens 80°C, wahlweise auch bis auf knapp unter 100°C zu erwärmen. Hierdurch kann die Autarkie bzw. Autonomie noch verbessert werden.

Die Heizeinrichtung kann zumindest teilweise auch am und/oder im Reservoir angeordnet sein. Die Heizeinrichtung kann z.B. wenigstens einen Heizstab oder Heizwendel aufweisen. Die Heizeinrichtung kann zumindest teilweise auch an und/oder in der Speiseleitung angeordnet sein.

In vorteilhafter Weise weist die Heizeinrichtung wenigstens zwei Heizelemente auf, welche sich im Reservoir erstrecken. Jedes Heizelement kann einen eigenen Stromanschluss aufweisen, insbesondere für 230V und/oder 400V. Somit kann je nach Stromversorgung vor Ort mit einer oder beiden oder mehreren Heizelementen Heizenergie eingetragen werden. Hierdurch kann nicht zuletzt Zeit eingespart werden.

Die Heizeinrichtung kann eingerichtet sein, ein Volumen von z.B. 20L bis 60L Wasser in wenigen Minuten aufzuheizen. Ein jeweiliges Heizelement kann eine Leistungsaufnahme von z.B. 3KW bis 12KW aufweisen.

Das Reservoir kann eine thermische Isolierung aufweisen, insbesondere zwecks effizientem Speichern von in das Spülmedium eingebrachter Wärmeenergie. Die Isolierung kann z.B. außen um das Reservoir herum angeordnet sein.

Die Desinfektionsvorrichtung kann eingerichtet sein, sämtliche Spülmedium führende Komponenten auf eine Mindest-Temperatur zu erhitzen, insbesondere mindestens 73°C, bevorzugt mindestens 80°C. Die Grenze von 80°C liefert einen guten Kompromiss aus thermischer Belastung der Armaturen und effizientem und zuverlässigem Erwärmen aller Komponenten auf mindestens 73°C. Die Mindest-Temperatur von ca. 80°C ist dabei auch bei einer rein thermischen Eigen-Desinfektion zweckdienlich.

Das Erhitzen auf die Mindest-Temperatur kann dabei wahlweise mittels Spülmedium oder auch ohne Spülmedium allein basierend auf Wärmeleitung oder Wärmestrahlung erfolgen. Die Heizeinrichtung kann derart dimensioniert und angeordnet sein, dass sämtliche Spülmedium führende Komponenten auf eine Mindest-Temperatur erwärmbar sind, insbesondere mindestens 73°C. Hierdurch kann Keimfreiheit mit hoher Sicherheit sichergestellt werden. Bevorzugt erfolgt das Erhitzen der Komponenten auf über 73°C bei einer Eigen-Desinfektion mittels Spülmedium in der Gasphase, insbesondere Wasserdampf.

Gemäß einem Ausführungsbeispiel ist die Desinfektionsvorrichtung eingerichtet, das Desinfizieren ausschließlich durch thermische Desinfektion durchzuführen, insbesondere gegen (Myko-)Bakterien, Keime und Verschmutzungen bei Temperaturen von mindestens 73°C, insbesondere auch mittels Spülmedium in der Gasphase. Es hat sich gezeigt, dass eine Temperatur über 72°C, wenn nicht alle, dann doch die meisten Bakterien vernichten kann, insbesondere wenn die Temperatur länger als 10min. gehalten wird. Die Desinfektionsvorrichtung kann eingerichtet sein, einen Desinfektionsgrad gemäß A0 600 sicherzustellen.

Es hat sich gezeigt, dass eine Desinfizierung mittels (Wasser-)Dampf oder Lufteinschlüssen bzw. Spülmedium in der Gasphase effektiver ist oder effizienter durchgeführt werden kann. Lufteinschlüsse können insbesondere für Verwirbelungen sorgen und z.B. einen Biofilm effizienter angreifen als ausschließlich in der Flüssigphase vorliegendes Spülmedium.

Gemäß einem Ausführungsbeispiel ist die Desinfektionsvorrichtung zur rein thermischen Eigen-Desinfektion, und insbesondere auch zur Eigendiagnose eingerichtet. Als rein thermische Eigen-Desinfektion ist dabei eine Desinfektion der Desinfektionsvorrichtung durch sich selbst zu verstehen, insbesondere ebenfalls spülmedienautonom. Die rein thermische Eigen-Desinfektion kann insbesondere mittels Spülmedium in der Gasphase durchgeführt werden. Als Eigendiagnose ist dabei eine Kontroll- oder Regelschleife zu verstehen, mittels welcher die Desinfektionsvorrichtung den eigenen Desinfektionsgrad überprüfen kann (Selbsttest). Die Eigendiagnose kann dabei das Sperren der Desinfektionsvorrichtung oder zumindest das Blockieren von Anschlüssen oder eines Desinfektionsvorgangs umfassen.

Gemäß einer Variante kann im Nachgang zu einer Eigen-Desinfektion eine Probe des gebrauchten Spülmediums bereitgestellt werden, insbesondere vollautomatisch durch Abfüllen eines Probenahme-Behälters, z.B. mit einem Volumen von 0.5L. Dies ermöglicht eine optionale Kontrolle durch ein unabhängiges Prüf-Labor. Derartige Labore sind häufig in Kliniken angesiedelt, so dass der Desinfektionsvorrichtung auch auf einfache Weise von einer externen Stelle ein bestimmter Desinfektions- bzw. Sterilitäts-Grad bescheinigt werden kann. Der Probenahme-Behälter kann dabei z.B. direkt mit dem Reservoir und/oder einem Kaltbehälter oder Rücklaufbehälter oder einer weiteren Komponente oder Leitung verbunden sein. Die Desinfektionsvorrichtung kann auch mehrere Probenahme-Behälter umfassen, insbesondere zur vollautomatischen Probenahme sowohl beim Desinfizieren ortsfester Leitungen als auch bei einer Eigen-Desinfektion. Dies ermöglicht, die Probenahme-Behälter zusammen zum Labor zu schicken, z.B. nachdem mehrere ortsfeste Leitungen desinfiziert wurden oder mehrere Eigen-Desinfektionen erfolgten.

Gemäß einem Ausführungsbeispiel umfasst die Desinfektionsvorrichtung ferner einen mit dem Reservoir verbundenen Kaltbehälter, insbesondere Kaltwasserbehälter, wobei der Kaltbehälter mittels eines Zuleit-Bypass, insbesondere beidseitig offenen Rohrs, mit dem Reservoir verbunden ist, welcher Zuleit-Bypass zwei freie Enden aufweist, welche in den Kaltbehälter und in das Reservoir hineinragen, insbesondere bis zu einer vordefinierten Füllstands-Höhe. Diese Anordnung erleichtert insbesondere auch eine Eigen-Desinfektion.

Der Kaltbehälter kann Spülmedium mit einer im Vergleich zum Reservoir deutlich kühleren Temperatur bereitstellen, z.B. mit Raumtemperatur. Der Kaltbehälter kann dabei vergleichsweise voluminös ausgebildet sein und z.B. ein Volumen im Bereich von 40 oder 50Litern aufweisen. Der Kaltbehälter kann auf derselben Höhe wie das Reservoir angeordnet sein, insbesondere jeweils bezüglich des Behälterbodens. Dies kann eine vorteilhafte, kompakte Anordnung aller Behälter ermöglichen und das Entleeren der Behälter erleichtern.

Mittels des Kaltbehälters kann ein Spülen der ortsfesten Leitung vor und/oder nach einem Schritte des Desinfizierens erfolgen, insbesondere zwecks Abkühlen der Leitung. Ebenso können Leitungen oder Komponenten der Desinfektionsvorrichtung umspült und abgekühlt werden.

Der Kaltbehälter kann sowohl mittels einer oder mehreren schaltbaren Leitungen als auch mittels einer ständig offenen Leitung (Zuleit-Bypass) mit dem Reservoir verbunden sein. Ein offener Bypass kann eine Eigen-Desinfektion ohne aufwändige Schaltungstechnik sicherstellen, insbesondere mittels Spülmedium in der Gasphase.

Der Kaltbehälter kann wahlweise auch mit einem/dem Rücklaufbehälter verbunden sein. Der Rücklaufbehälter kann dabei ein Volumen entsprechend mindestens der Summe der Volumina von Reservoir und Kaltbehälter aufweisen. Somit kann der gesamte Inhalt von Reservoir und Kaltbehälter vom Rücklaufbehälter aufgenommen werden. Dies verbessert die Autarkie bzw. Autonomie der Desinfektionsvorrichtung.

In vorteilhafter Weise ist der Kaltbehälter neben dem Reservoir und/oder über dem Rücklaufbehälter angeordnet. Durch diese Anordnung können sowohl das Reservoir als auch der Kaltbehälter ohne Pumparbeit nur über natürliches Gefälle in den Rückwasserbehälter entleert werden. In vorteilhafter Weise ist der Rücklaufbehälter unter dem Reservoir und/oder unter dem Kaltbehälter angeordnet. Dies minimiert nicht zuletzt eine erforderliche Förderleistung.

In vorteilhafter Weise ist der Kaltbehälter mit dem Reservoir und dem Rücklaufbehälter verbunden, insbesondere jeweils über ventillose, beidseitig offene Rohre. Dies ermöglicht nicht zuletzt auch eine effektive oder effiziente Geräteaufbereitung, insbesondere mittels (Wasser-)Dampf bzw. Spülmedium in der Gasphase. Eine Rohrverbindung zwischen dem Reservoir und dem Kaltbehälter kann derart ausgebildet sein, dass im normalen Desinfektionsbetrieb beim Spülen von ortsfesten Leitungen kein Gasaustausch zwischen diesen Behältern stattfinden kann. Eine Rohrverbindung zwischen dem Kaltbehälter und dem Rückwasserbehälter kann derart ausgebildet sein, dass auch eine Funktion als Überlauf von Spülmedium zum Rücklaufbehälter erfüllt werden kann.

Die Desinfektionsvorrichtung kann einen Rollwagen aufweisen, auf welchem das Reservoir und die beiden Behälter angeordnet sind und zusammen verlagerbar sind.

Das Reservoir und der jeweilige Behälter können jeweils einen Abfluss aufweisen. Das Reservoir und der jeweilige Behälter können jeweils Ventile an Zu- und Ableitungen sowie wenigstens einen Temperatursensor sowie wenigstens einen Füllstandsensor aufweisen.

Wie zuvor beschrieben, kann der Zuleit-Bypass zwei freie Enden aufweisen. Im Kaltbehälter kann das freie Ende in Spülmedium in der Flüssigphase stehen, insbesondere in Trinkwasser, insbesondere zumindest während dem Desinfizieren einer ortsfesten Leitung. Anders ausgedrückt: Im Kaltbehälter kann das freie Ende auf einer Höhe angeordnet sein, die unter dem Füllstand im Kaltbehälter liegt, und wahlweise auch tiefer als das freie Ende im Reservoir. Dies ermöglicht eine hydrostatische Sperre zwischen dem Reservoir und dem Kaltbehälter, insbesondere während dem Desinfektionsvorgang an der ortsfesten Leitung. Insbesondere kann dadurch vermieden werden, dass Spülmedium in der Gasphase in den Kaltbehälter übertritt.

Der Zuleit-Bypass kann das Reservoir mit dem Kaltbehälter in Reihe schalten, insbesondere zwecks gemeinsamer Desinfektion mittels Spülmedium in der Gasphase. Spülmedium in der Gasphase kann durch diese Komponenten und damit verbundene Armaturen geführt werden, insbesondere ohne das Erfordernis, einen Überdruck aufzubauen. Vielmehr kann eine kleine Druckdifferenz im Bereich von wenigen Millibar, z.B. 10mbar ausreichen.

Beim Desinfizieren der ortsfesten Leitung ermöglicht der Zuleit-Bypass einen Sicherheitsmechanismus. Zwar ist der Zuleit-Bypass derart angeordnet und geformt, dass flüssiges Spülmedium nicht über den Zuleit-Bypass aus dem Reservoir entweichen kann. Jedoch kann ein Druckübergang stattfinden, beispielsweise für den Fall dass eine Phasenumwandlung stattfindet und sich gasförmiges Spülmedium bildet. Somit kann auch ein zuverlässiger, risikoarmer Betrieb bei vergleichsweise hohen Temperaturen erfolgen (insbesondere bei über 90°C oder sogar 95°C, sofern gewünscht). Der Zuleit-Bypass kann verhindern, dass sich im Reservoir ein Überdruck aufbaut. Der Zuleit-Bypass kann dabei beidseitig offen bleiben. Der Zuleit-Bypass kann sich höher als eine maximale Füllstand-Höhe im Reservoir erstrecken. Dies kann sicherstellen, dass flüssiges Spülmedium nicht vom Reservoir in den Kaltbehälter gelangen kann, sondern allenfalls gasförmiges Spülmedium.

Der Zuleit-Bypass kann auch zum Weiterleiten von Spülmedium dienen, insbesondere Spülmedium in der Gasphase zum Aufbereiten bzw. Desinfizieren der Desinfektionsvorrichtung. Es hat sich gezeigt, dass mittels des Zuleit-Bypass ein Desinfizieren auf besonders zuverlässige und effiziente Weise erfolgen kann, insbesondere ohne das Erfordernis, Spülmedium mittels Düsen in bestimmte Ecken oder Winkel der Spülmittel führenden Komponenten der Desinfektionsvorrichtung leiten zu müssen.

Gemäß einem Ausführungsbeispiel ist der Zuleit-Bypass beidseitig offen und ventillos ohne Ventile ausgebildet. Es hat sich gezeigt, dass dieser vergleichsweise simple Aufbau viele vorrichtungstechnische und verfahrenstechnische Vorteile liefert. Armaturen und Steuerungstechnik kann eingespart werden. Hierdurch kann auch gänzlich ausgeschlossen werden, dass sich beim Erhitzen des Spülmediums im Reservoir ein Druck im Reservoir aufbaut. Reservoir und Kaltbehälter müssen also nicht notwendigerweise als Druckbehälter ausgelegt bzw. geprüft oder zugelassen werden. Dampf bzw. Spülmedium in der Gasphase kann durch den Zuleit-Bypass bzw. das Rohr entweichen. Ein ventilloser, offener Zuleit-Bypass liefert eine robuste Anordnung, welche mittels einer einfachen Steuerung betrieben werden kann.

Gemäß einem Ausführungsbeispiel erstreckt sich der Zuleit-Bypass U-förmig mit beidseitig nach unten offenen Enden. Dies ermöglicht das Einleiten von Spülmedium in der Gasphase derart dass der Kaltbehälter von unten nach oben vom Spülmedium umströmt bzw. durchströmt wird. Auch kann dadurch vermieden werden, dass sich im Zuleit-Bypass Kondensat sammelt.

Gemäß einem Ausführungsbeispiel überlappt ein sich im Kaltbehälter vertikal erstreckendes freies Ende des Zuleit-Bypass ein sich im Reservoir vertikal erstreckendes freies Ende des Ableit-Bypass in vertikaler Richtung. Dies kann sicherstellen, dass das Spülmedium den Kaltbehälter möglichst vollständig durchströmt. Gleichzeitig können Kaltbehälter und Reservoir zumindest annähernd in derselben Höhe angeordnet sein. Die überlappende Anordnung liefert Vorteile hinsichtlich einer vollständigen Durchströmung des Kaltbehälters und hinsichtlich der Anordnung der Behälter relativ zueinander.

Gemäß einem Ausführungsbeispiel erstreckt sich das freie Ende des Zuleit-Bypass im Kaltbehälter tiefer bzw. weiter nach unten als das freie Ende des Zuleit-Bypass im Reservoir. Dies erleichtert das Einstellen einer hydrostatischen Sperre. Indem das offene Ende des Zuleit-Bypass im Kaltbehälter vergleichsweise nah am Boden angeordnet ist, kann bereits eine kleine Menge Spülmedium im Kaltbehälter ausreichen, um einen Austausch zwischen dem Reservoir und dem Kaltbehälter zu verhindern. Je höher der Füllstand im Kaltbehälter, desto größer ist ein Gegendruck.

Gemäß einem Ausführungsbeispiel weist die Desinfektionsvorrichtung einen Rücklaufbehälter auf, wobei der Kaltbehälter mittels eines Ableit-Bypass, insbesondere beidseitig offenen Rohrs, mit dem Rücklaufbehälter verbunden ist, welcher Ableit-Bypass zwei freie Enden aufweist, welche in den Kaltbehälter und in den Rücklaufbehälter hineinragen, insbesondere jeweils bis zu einer vordefinierten Füllstands-Höhe. Der Ableit-Bypass ermöglicht einen hindernisfreien Gasaustausch zwischen dem Kaltbehälter und dem Rücklaufbehälter. Der Ableit-Bypass kann beide Behälter in Reihe schalten, insbesondere hinter das Reservoir. Beim Desinfizieren der ortsfesten Leitung ermöglicht der Ableit-Bypass auch einen Überlauf vom Kaltbehälter in den Rücklaufbehälter.

Gemäß einem Ausführungsbeispiel weist die Desinfektionsvorrichtung einen Rücklaufbehälter und einen Kaltbehälter sowie einen Zuleit-Bypass und einen Ableit-Bypass auf, welche Bypässe eine offene Reihenschaltung des Reservoirs mit den beiden Behältern bilden. Dies ermöglicht das Desinfizieren von Reservoir und beider Behälter auf einfache Weise, insbesondere indem Spülmedium in der Gasphase durch die offene Reihenschaltung geführt wird. Das Desinfizieren kann dabei auch besonders effizient ausgeführt werden, insbesondere in zeitlicher Hinsicht. Trotz voluminöser Behälter kann die Anordnung in wenigen Minuten auf z.B. über 80°C erhitzt werden.

Das erste Ende des Ableit-Bypass kann oberhalb vom Zuleit-Bypass angeordnet sein, insbesondere in einem oberen Dritte des Kaltbehälters. Der Zuleit-Bypass und der Ableit-Bypass können sich im Kaltbehälter über eine möglichst große Länge überlappen. Dies kann einen vergleichsweise langen Strömungspfad von Spülmedium in der Gasphase sicherstellen.

Gemäß einem Ausführungsbeispiel ist die Desinfektionsvorrichtung eingerichtet, sich selbst automatisch zu desinfizieren (Eigen-Desinfektion), indem das Reservoir mit einem oder mehreren weiteren Behältern in einer offenen Reihenschaltung mittels Spülmedium in der Gasphase durchspült wird, insbesondere ohne Ventilschaltung in der Reihenschaltung. Das Spülmedium kann beispielsweise am Ende der Reihenschaltung, insbesondere im Rücklaufbehälter kondensieren und dann abgeleitet werden. Auch kann durch automatisches Umschalten von Ventilen in der Anlage sichergestellt werden, dass alle Behälter, Rohrleitungen und Armaturen des Desinfektionsgerätes desinfiziert werden.

Gemäß einem Ausführungsbeispiel ist die Desinfektionsvorrichtung als mobiles, verlagerbares Standgerät ausgebildet. Wie zuvor beschrieben, erleichtert eine verlagerbare Desinfektionsvorrichtung die Handhabung und erweitert die Einsatzmöglichkeiten und verstärkt die Autonomie. Das Verlagern kann motorisch zumindest unterstützt werden.

Gemäß einem Ausführungsbeispiel weist die Desinfektionsvorrichtung einen Druckluftanschluss auf und ist eingerichtet, Druckluft ins Spülmedium und/oder die jeweilige ortsfeste Leitung oder die Spülleitung einzubringen.

Gemäß einem Ausführungsbeispiel umfasst die Messeinrichtung wenigstens einen an die Steuerungseinrichtung gekoppelten Durchflusssensor zum Erfassen einer Durchflussmenge des Spülmediums an einer jeweiligen ortsfesten Leitung und/oder in der Speiseleitung und/oder im Rücklauf, und/der eine mit der Steuerungseinrichtung gekoppelte Zeiterfassungseinheit. Ein jeweiliger Durchflusssensor kann dabei auch für eine Bilanz des Spülmediums genutzt werden, insbesondere indem die Durchflussmenge von wenigstens zwei Durchflusssensoren kontinuierlich aufgezeichnet und verglichen wird. Dies ermöglicht eine Überwachung hinsichtlich Leitungsbruch oder einer Leckage.

Gemäß einem Ausführungsbeispiel sind wenigstens zwei Durchflusssensoren vorgesehen, die sowohl vor (stromauf) als auch hinter (stromab) dem Reservoir angeordnet sind. Dies ermöglicht, Lecks zu erkennen. Die Steuerungseinrichtung ist eingerichtet, einen Differenzwert zwischen der von diesen Durchflusssensoren erfassten Durchflussrate zu erfassen und abzuspeichern, insbesondere zum Erstellen einer Durchfluss-Bilanz. Die Steuerungseinrichtung ist eingerichtet, bei einem Differenzwert über einem Schwellwert einen Desinfektionsvorgang zu unterbrechen.

Gemäß einem Ausführungsbeispiel ist die Messeinrichtung zum autonomen Erfassen von geografischen Daten bzw. des jeweiligen Einsatzortes (Standortes) eingerichtet, insbesondere mittels einer Ausleseeinheit. Dank automatischer Standorterfassung kann eine Fehleingabe oder Manipulation des Desinfektionsprotokolls ausgeschlossen werden. Dabei kann die Steuerungseinrichtung zum Steuern oder Regeln eines Desinfektionsvorgangs in Abhängigkeit von in der Steuerungseinrichtung hinterlegten und/oder von einem Bediener eingegebenen ortsspezifischen Daten eingerichtet sein, insbesondere Einsatzort, Leitungsvolumen, Leitungslänge. Dies erleichtert die exakte Aufzeichnung und Dokumentation von ortsbezogenen Desinfektionsdaten, insbesondere mit Bezug zu einem jeweiligen Operationssaal eines Krankenhauses. Die Ausleseeinheit kann beispielsweise auch ein GPS zum autonomen Erfassen von absoluten geodätischen Daten umfassen. Das Erfassen von geografischen Daten bzw. Ortsangaben kann beispielsweise auch durch Auslesen eines Chip erfolgen, welcher in einem jeweiligen Vorraum bzw. in unmittelbarer Nähe eines Operationssaals installiert ist. Im Chip können nicht nur Ortsangaben bzw. Raum-Informationen hinterlegt sein, sondern z.B. auch Angaben über die Länge oder das Volumen einer jeweiligen ortsfesten Leitung.

Es hat sich gezeigt, dass das autonome Auslesen von am jeweiligen Einsatzort hinterlegten Daten vorteilhafter ist als z.B. die Verwendung eines barcode-Scanners. Zum einen wird die Handhabung erleichtert, zum anderen können Fehlerquellen vermieden werden. Die Ausleseeinheit kann hierfür eine Sende-/Empfangseinheit mit vorgegebener Reichweite aufweisen, insbesondere einige Zentimeter oder Meter, so dass nur die am Einsatzort hinterlegten Daten ausgelesen werden. Die Reichweite kann einstellbar sein, insbesondere um die Handhabung zu erleichtern und die Reichweite an den Abstand ortsfester Datenträger/Chips zueinander anzupassen. Dank einstellbarer Reichweite kann das Risiko von Fehlern beim Auslesen minimiert werden, ohne die Handhabung bzw. den Aktionsradius der Desinfektionsvorrichtung unnötig einzuschränken. Nichtsdestotrotz kann die Desinfektionsvorrichtung auch einen an die Steuerungsvorrichtung gekoppelten Barcode-Scanner umfassen.

Gemäß einem Ausführungsbeispiel ist die Steuerungseinrichtung zum Erstellen und Abspeichern eines Desinfektions-Protokolls bzw. Prüfberichts basierend auf erfassten Messwerten eingerichtet. Dabei kann die Desinfektionsvorrichtung eine Anzeige- oder Ausgabeeinheit zum Anzeigen oder Ausgeben eines Desinfektions-Protokolls aufweisen, insbesondere in Papierform zur visuellen Dokumentation am Einsatzort der Desinfektionsvorrichtung. Dabei kann auch ein Zustands-Bericht ausgegeben werden, bevor die Desinfektionsvorrichtung den Desinfektionsvorgang beginnt. Die Steuerungseinrichtung ist dann eingerichtet, vor dem Beginn der Desinfektion ortsfester Leitungen den Zustand der Desinfektionsvorrichtung zu überprüfen und einen Zustands-Bericht auszugeben oder zumindest zusammen mit einem Desinfektions-Protokoll abzuspeichern oder auszugeben. Falls die Desinfektionsvorrichtung nicht oder nicht ordnungsgemäß desinfiziert wurde, oder falls der Desinfektionsvorgang schon eine Mindest-Dauer größer einem vorgebbaren zeitlichen Schwellwert zurückliegt, kann eine Warnmeldung ausgegeben werden und/oder das Verfahren zum Desinfizieren ortsfester Leitungen blockiert werden. Das Desinfektions-Verfahren kann dann blockiert werden, bis die Desinfektionsvorrichtung selbst (erneut) desinfiziert wurde, insbesondere gemäß dem hier beschriebenen Aufbereitungs-Verfahren. Diese Selbst-Überwachung bzw. Eigendiagnose erhöht die Sicherheit und verringert das Risiko, Bakterien oder Keime mittels der Desinfektionsvorrichtung in die ortsfesten Leitungen einzutragen.

ITEM Die zuvor genannte Aufgabe wird auch gelöst durch eine Desinfektionsvorrichtung zum thermischen Desinfizieren von ortsfesten Leitungen, mit: einem Reservoir für Spülmedium, zum Desinfizieren einer jeweiligen ortsfesten Leitung; wenigstens einer vom Reservoir abgehenden Speiseleitung mit einem Auslass zum Kuppeln der Desinfektionsvorrichtung an eine jeweilige ortsfeste Leitung; einer Heizeinrichtung zum Bereitstellen des Spülmediums mit einer bezüglich Raumtemperatur erhöhten Soll-Temperatur, insbesondere mindestens 73°C; einer mit der Heizeinrichtung gekoppelten Steuerungseinrichtung zum Einstellen, Steuern und/oder Regeln einer Temperatur des Spülmediums; wenigstens einer an die Steuerungseinrichtung gekoppelten Messeinrichtung zum Erfassen wenigstens einer Messgröße des Spülmediums, insbesondere einer Temperatur; einem Rücklauf zum Zurückführen von Spülmedium aus der ortsfesten Leitung zurück in die Desinfektionsvorrichtung mit einem Einlass zum Kuppeln der jeweiligen ortsfesten Leitung an den Rücklauf; wobei die Desinfektionsvorrichtung spülmediumautonom ist, wobei die Desinfektionsvorrichtung eingerichtet ist, mittels der Speiseleitung und dem Rücklauf in Verbindung mit einer jeweiligen ortsfesten Leitung einen bezüglich der Desinfektionsvorrichtung auch extern angeordneten desinfizierbaren, hermetischen Umlauf zu bilden, wobei das Reservoir ein Heißreservoir zum Bereitstellen und Vorhalten von Spülmedium mit einer Temperatur von mindestens 73°C ist, wobei die Desinfektionsvorrichtung zur rein thermischen Eigen-Desinfektion, und insbesondere auch zur Eigendiagnose eingerichtet ist, wobei die Desinfektionsvorrichtung einem mit dem Reservoir verbundenen Kaltbehälter aufweist, wobei der Kaltbehälter mittels eines Zuleit-Bypass, insbesondere beidseitig offenen Rohrs, mit dem Reservoir verbunden ist, welcher Zuleit-Bypass zwei freie Enden aufweist, welche in den Kaltbehälter und in das Reservoir hineinragen, insbesondere bis zu einer vordefinierten Füllstands-Höhe, wobei der Zuleit-Bypass beidseitig offen und ventillos ohne Ventile ausgebildet ist, wobei die Desinfektionsvorrichtung als mobiles Gerät ausgebildet ist, und wobei das Reservoir ein Volumen für Spülmedium entsprechend einem Vielfachen, insbesondere mindestens Faktor 3 bis 5, des Volumens der jeweiligen ortsfesten Leitung bereitstellt. Hierdurch ergeben sich viele der zuvor genannten Vorteile.

Die Desinfektionsvorrichtung kann zusammen mit zwei, drei oder noch mehr Verbindungsschläuchen bereitgestellt werden, welche ein Kuppeln der Desinfektionsvorrichtung an eine jeweilige ortsfeste Leitung erleichtern. Ein derartiger Verbindungsschlauch, z.B. aus Silikon, kann dabei auch zum Kurzschließen oder Verbinden von zwei ortsfesten Leitungen verwendet werden. Wahlweise kann auch ein Ende der Speiseleitung oder ein Anfang des Rücklaufs (bzw. der Einlass) in der Art eines flexiblen Schlauches mit Kupplung ausgebildet sein.

Die zuvor genannte Aufgabe wird auch gelöst durch Verwendung einer spülmediumautonomen Desinfektionsvorrichtung, insbesondere einer hier beschriebenen Desinfektionsvorrichtung, zum thermischen Desinfizieren von Innenflächen von ortsfesten Leitungen. Dazu kann ein Reservoir mit einem Volumen an Spülmedium mit einem Vielfachen des Volumens der zu desinfizierenden ortsfesten Leitung bereitgestellt werden. Hierdurch ergeben sich bereits zuvor beschriebene Vorteile.

Die zuvor genannte Aufgabe wird auch gelöst durch Verwendung einer Desinfektionsvorrichtung, insbesondere einer hier beschriebenen Desinfektionsvorrichtung, zum rein thermischen Desinfizieren von ortsfesten Leitungen, wobei sich die Desinfektionsvorrichtung vor oder nach einem Desinfektionsvorgang automatisch auf rein thermische Weise spülmediumautonom selbst aufbereitet bzw. desinfiziert (autonome Eigen-Desinfektion). Hierdurch ergeben sich bereits zuvor beschriebene Vorteile. Dabei kann auch jeweils eine automatische Probenahme in wenigstens einen Probenahme-Behälter der Desinfektionsvorrichtung erfolgen.

Die zuvor genannte Aufgabe wird auch gelöst durch Verwendung einer spülmediumautonomen Desinfektionsvorrichtung zum Desinfizieren von Innenflächen von ortsfesten medizintechnischen Leitungen für Hypothermieanwendungen gegen Mykobakterien, insbesondere einer hier beschriebenen Desinfektionsvorrichtung, wobei die Desinfektionsvorrichtung von extern ausschließlich mit Energie versorgt wird. Hierdurch ergeben sich bereits zuvor beschriebene Vorteile.

Die zuvor genannte Aufgabe wird auch gelöst durch Verwendung einer spülmediumautonomen Desinfektionsvorrichtung zum thermischen Desinfizieren von Innenflächen von medizintechnischen Leitungen für Hypothermieanwendungen gegen Mykobakterien, insbesondere einer hier beschriebenen Desinfektionsvorrichtung, in einem Vorraum zu einem Operationssaal zum Desinfizieren von in den Operationssaal führenden Leitungen, wobei Spülmedium ausschließlich einem Reservoir der Desinfektionsvorrichtung entnommen wird und zur Desinfektionsvorrichtung zurückgeführt wird. Hierbei kann ein Umlauf für das Spülmedium bereitgestellt werden. Das Spülmedium kann wahlweise in nur einer Schleife und/oder im Kreis geführt werden. Das Spülmedium kann zwischengelagert bzw. gepuffert werden.

Die zuvor genannte Aufgabe wird auch gelöst durch Verwendung wenigstens eines beidseitig offenen Bypass jeweils zwischen zwei Spülmedium aufnehmenden Behältern einer Desinfektionsvorrichtung zum Desinfizieren von ortsfesten Leitungen, insbesondere zwei Bypass-Rohrleitungen zur Reihenschaltung von drei Behältern, zum Aufbereiten bzw. Desinfizieren der Desinfektionsvorrichtung mittels Spülmedium in der Gasphase (autonome Eigen-Desinfektion), insbesondere in einer hier beschriebenen Desinfektionsvorrichtung. Hierdurch ergeben sich bereits zuvor beschriebene Vorteile.

Die zuvor genannte Aufgabe wird auch gelöst durch ein Verfahren zum automatisierten Desinfizieren von Innenflächen von ortsfesten Leitungen, insbesondere medizintechnischen Leitungen, insbesondere durch Verwendung einer hier beschriebenen Desinfektionsvorrichtung, mit den Schritten:
- Einstellen wenigstens eines Wertes wenigstens einer Messgröße des Spülmediums oder wenigstens eines Verfahrensparameters, insbesondere eines Wertes für eine Soll-Temperatur und/oder einer Mindest-Spüldauer zum Desinfizieren der (jeweiligen) ortsfesten Leitung, oder Abrufen des wenigstens einen Wertes aus einem Speicher der Desinfektionsvorrichtung, insbesondere eines Wertes abhängig vom Einsatzort der Desinfektionsvorrichtung, z.B. das Innenvolumen der zu desinfizierenden ortsfesten Leitung;
- Erfassen wenigstens eines Messwertes und Regeln wenigstens einer Messgröße des Spülmediums, insbesondere zumindest der Temperatur des Spülmediums im Reservoir;
- spülmediumautonomes Desinfizieren durch automatisiertes Einleiten von Spülmedium aus dem Reservoir in die ortsfeste Leitung und Rückführen des eingeleiteten Spülmediums aus der ortsfesten Leitung zurück in die Desinfektionsvorrichtung;
- Aufzeichnen und Abspeichern von erfassten Messwerten, insbesondere von Ist-Temperaturen des Spülmediums an einem Rücklauf der Desinfektionsvorrichtung oder von der Dauer des Spülens.

Vor dem Einstellen oder Abrufen von Messgrößen bzw. Messwerten können auch folgende Schritte durchgeführt werden, sei es manuell, sei es automatisch bzw. motorisch:
- Bereitstellen einer Desinfektionsvorrichtung mit einem Reservoir beinhaltend Spülmedium an wenigstens einer ortsfesten Leitung;
- Anschließen sowohl wenigstens eines Auslasses als auch wenigstens eines Einlasses der Desinfektionsvorrichtung an die (jeweilige) ortsfeste Leitung.

Das Desinfizieren von Leitungen umfasst das Desinfizieren von Innenoberflächen bzw. von langgestreckten engen Hohlräumen mit einem großen Längen-Breiten-Verhältnis.

In vorteilhafter Weise wird eine jeweilige ortsfeste Leitung jeweils separat desinfiziert. Mehrere ortsfeste Leitungen werden dann chronologisch nacheinander desinfiziert. Somit kann für jede Leitung eine durchgeführte Desinfektion bescheinigt werden. Es besteht kein Risiko, dass Messergebnisse falsch zugeordnet werden. Wahlweise können mehrere ortsfeste Leitungen auch miteinander in Reihe verkuppelt werden, um diese zusammen zu desinfizieren. Dabei wird jedoch auch nur ein Umlauf gebildet: Das Spülmedium durchläuft alle Leitungen, bevor es in den Rücklaufbehälter geleitet wird.

Gemäß einer Variante können auch mehrere separate ortsfeste Leitungen desinfiziert werden. Dabei ist jede ortsfeste Leitung jeweils an einem Auslass und Einlass der Desinfektionsvorrichtung angeschlossen. Anders ausgedrückt: Für jede ortsfeste Leitung weist die Desinfektionsvorrichtung wenigstens einen Auslass und einen Einlass auf. Ferner kann für jede ortsfeste Leitung bzw. jeden dafür vorgesehenen Umlauf eine separate Sensorik vorgesehen sein. Dies ermöglicht zeiteffizientes, synchrones Desinfizieren von mehreren Leitungen, wobei für jede der Leitungen jeweils ein spezifisches Desinfektionsprogramm durchlaufen werden kann. In der Praxis kann dies zu großen Zeiteinsparungen führen, insbesondere weil auf Abkühlphasen verzichtet werden kann.

Das Anschließen kann beispielsweise mittels beidseitig abdichtenden, genormten Kupplungen erfolgen, insbesondere auf fluiddichte Weise.

Vor oder nach dem Anschließen kann ein Aktivieren der Desinfektionsvorrichtung durch Zuführen von elektrischer Energie erfolgen. Das Zuführen von elektrischer Energie kann aus einem internen Energiespeicher und/oder über eine externe Stromleitung erfolgen.

Das Desinfizieren kann das Ausführen eines Desinfektionsvorgangs oder Desinfektionsprogramms umfassen. Ein jeweiliges Desinfektionsprogramm kann in einem Daten- oder Programmspeicher der Desinfektionsvorrichtung hinterlegt sein. Das Desinfizieren oder Spülen kann dabei auch ein statisches Verweilen von Spülmedium in der Leitung umfassen, insbesondere während einer vorgegebenen Zeitspanne.

Das Einleiten und Rückführen kann jeweils derart erfolgen, dass das Spülmedium nicht im Kreis geführt wird, sondern lediglich einmalig durch die Leitung geführt wird. Dies kann auch wiederholt mit vordefinierten Volumina erfolgen, wobei die Volumina in Abhängigkeit des zu spülenden Leitungsvolumens vorgegeben werden können, beispielsweise dem 1.5fachen Volumen. Dabei kann je Spülzyklus auch eine Mindest-Verweilzeit vorgegeben werden, sei es bei Durchströmung, sei es in statischem Zustand mit einem Volumenstrom gleich NULL.

Als Spüldauer ist dabei insbesondere eine Verweilzeit von Spülmedium in der jeweiligen ortsfesten Leitung zu verstehen. Wahlweise kann auch eine Durchflussmenge aufgezeichnet und geregelt werden. Das Aufzeichnen von Messwerten kann dabei das Erfassen der Messwerte und Weiterleiten an eine Steuerungseinrichtung umfassen.

Das Verfahren des Desinfizierens kann durch Entkoppeln der Desinfektionsvorrichtung vom Einlass bzw. Auslass beendet werden. Es können beidseitig abdichtende Kupplungen verwendet werden, so dass vorheriges Abpumpen der ortsfesten Leitung nicht erforderlich ist. Die Leitung kann befüllt bleiben.

Gemäß einer Ausführungsform umfasst der Schritt des Erfassens und Regelns das Erfassen einer Temperatur am Einlass und am Rücklauf sowie eines Durchflusses durch die Speiseleitung sowie das Regeln eines Durchflusses durch die Speiseleitung, insbesondere derart, dass die Temperatur am Rücklauf größer gleich 73°C ist und eine Differenz zwischen der Temperatur am Einlass und der Temperatur am Rücklauf kleiner 5°C ist. Eine derartige vergleichsweise kleine Differenz kann sicherstellen, dass alle Leitungsabschnitte im Wesentlichen mit derselben Energie beaufschlagt wurden. Der Desinfektions-Vorgang wird dadurch vergleichsweise homogen durchgeführt, insbesondere auch auf sehr kontrollierte Art und Weise. Dies minimiert nicht zuletzt auch die thermische Belastung der Armaturen.

Gemäß einer Ausführungsform wird in einem Reservoir der Desinfektionsvorrichtung ein Spülmedium-Volumen vorgehalten, welches mindestens um den Faktor 3, insbesondere um den Faktor 3 bis 5 größer ist als das Innenvolumen der zu desinfizierenden ortsfesten Leitung, wobei das Spülmedium auf eine Temperatur erhitzt ist/wird, welche größer als die für die Desinfektion einzuhaltende Mindest-Temperatur (Soll-Temperatur) ist. Die Temperatur-Differenz kann z.B. im Bereich von 5 bis 10°C liegen. Hierdurch kann sichergestellt werden, dass ins Reservoir zurückgeführtes Spülmedium das im Reservoir vorgehaltene Spülmedium nicht zu stark abkühlt. Ein vergleichsweise großer Energiepuffer ermöglicht das Halten der gewünschten Mindest-Temperatur (insbesondere 73°C), und zwar auch bei vergleichsweise großen Durchflussraten. Es hat sich gezeigt, dass ein um den Faktor 3 bis 5 größeres Volumen einen guten Kompromiss aus Handhabung der Vorrichtung und bereitgehaltenem Energieinhalt liefert.

Gemäß einer Ausführungsform erfolgt das Desinfizieren auf rein thermische Weise, insbesondere bei Soll-Temperaturen von mindestens 73°C, wobei ein Erstellen und Abspeichern eines Desinfektions-Protokolls bzw. Prüfberichts basierend auf erfassten Messwerten umfassend zumindest Temperaturwerte erfolgt. Die Ist-Temperatur wird dabei zumindest auch am Einlass bzw. im Rücklauf erfasst. Dabei kann die Ist-Temperatur insbesondere auch bezüglich des Rücklaufs aufgezeichnet, dokumentiert bzw. zur Überprüfung ausgegeben werden. Am Reservoir oder an einer Speiseleitung kann eine Temperatur höher als die Mindest-Temperatur eingestellt werden, insbesondre 1 bis 10°C höher. Die Temperatur an bzw. in der Speiseleitung kann durch Zuführen von Heizleistung oder Regeln des Durchflusses derart eingestellt und geregelt werden, dass am Rücklauf mindestens die Mindest-Temperatur vorliegt.

Als Spülmedium wird vorteilhafter Weise Wasser verwendet, insbesondere zusatzfreies Wasser mit Trinkwasserqualität. Dies ermöglicht, den ortsfesten, insbesondere medizintechnischen Leitungen nach erfolgter Desinfektion zumindest Reinheit gemäß Trinkwasserqualität zu bescheinigen. Als "zusatzfreies Wasser" ist Wasser zu verstehen, welchem zwecks Desinfizierung keine weiteren, insbesondere chemischen Mittel zugegeben wurden. Die Desinfektion kann ausschließlich auf thermische Weise erfolgen. Das Spülmedium kann ausschließlich in der Flüssigphase vorliegen oder auch teilweise in der Gasphase vorliegen. Das Spülmedium kann zumindest teilweise in der Gasphase vorliegen, also auch vollständig in der Gasphase, zumindest zeitweise während einzelner Zeitabschnitte eines Desinfektionsvorgangs. Indem mit der Gasphase gespült wird, kann das Desinfizieren auf vergleichsweise effiziente oder effektive Weise erfolgen. Gemäß einer Variante kann das genutzte Spülmedium auch vollständig in der Gasphase vorliegen, insbesondere zwecks Eigen-Desinfektion.

Gemäß einer Ausführungsform erfolgt das Desinfizieren mittels Spülmedium in Form von Wasser in der Flüssig- und/oder Gasphase, insbesondere zusatzfreiem Wasser mit zumindest Trinkwasserqualität, insbesondere mit zumindest zeitweise kontinuierlich anhaltendem Spülmedium-Volumenstrom. Das Desinfizieren kann mit Spülmedium mit einem bestimmten Volumenstrom erfolgen, insbesondere auch mit kontinuierlich anhaltendem konstantem Volumenstrom. Ein kontinuierlich anhaltender, beispielsweise konstanter Spülmedium-Volumenstrom kann eine gute Überwachung bzw. Kontrolle des Desinfektionsvorgangs ermöglichen.

Gemäß einer Variante wird der Volumenstrom für bestimmte Perioden des Desinfektionsvorgangs variiert. Dann erfolgt das Desinfizieren bei wenigstens zwei vordefinierten Volumenströmen.

Gemäß einer Ausführungsform umfasst Einleiten von Spülmedium zusätzlich auch ein Durchspülen der ortsfesten Leitung mit Spülmedium aus dem Reservoir oder aus einem Kaltbehälter der Desinfektionsvorrichtung, insbesondere vor und/oder nach dem Schritt Desinfizieren. Das Durchspülen kann dabei wahlweise bei Soll-Temperatur oder einer niedrigeren Temperatur erfolgen, insbesondere auch bei Raumtemperatur. Beim Durchspülen kann dabei auch die Durchflussmenge erfasst werden, insbesondere zum Bestimmen des Volumens der ortsfesten Leitung.

Gemäß einer Ausführungsform erfolgt das Durchspülen mit Spülmedium in Form von Frischwasser aus dem Kaltbehälter, wobei die ortsfeste Leitung vollständig mit Frischwasser gefüllt wird. Hierdurch kann ausgeschlossen werden, dass Luft in der ortsfesten Leitung verbleibt.

Gemäß einer Ausführungsform erfolgt nach Abschluss des Schrittes Desinfizieren eine Temperaturkontrolle an einem Rücklauf der Desinfektionsvorrichtung, wobei bei Unterschreiten einer Maximal-Temperatur ein mit dem Rücklauf verbundener Einlass der Desinfektionsvorrichtung freigegeben wird. Das Freigeben des Einlasses kann dabei auch einen Schutz vor Verbrühungen ermöglichen. Das Freigeben kann z.B. durch oder in Verbindung mit einem akustischen Signal erfolgen. Das Freigeben kann ein Entsperren einer am Einlass vorgesehenen Kupplung umfassen. Ein Freigeben erleichtert somit die Handhabung, auch für nicht geschulte Bediener der Desinfektionsvorrichtung.

Falls ortsfeste medizintechnische Leitungen in einer Herzklinik desinfiziert werden müssen, kann der Bediener auch ein Kardio-Techniker sein. Dieser ist üblicherweise geschult. Nichtsdestotrotz ist eine einfache Handhabung auch für den Kardio-Techniker vorteilhaft.

Die zuvor genannte Aufgabe wird auch gelöst durch ein Verfahren zum Aufbereiten einer Desinfektionsvorrichtung eingerichtet zum Desinfizieren von ortsfesten Leitungen, insbesondere einer hier beschriebenen Desinfektionsvorrichtung, insbesondere in Verbindung mit bzw. in Chronologie mit einem hier beschriebenen Verfahren zum Desinfizieren ortsfester Leitungen, wobei ein spülmediumautonomes rein thermisches Desinfizieren von Spülmedium führenden Komponenten der Desinfektionsvorrichtung mittels in einem Reservoir der Desinfektionsvorrichtung bereitgehaltenem Spülmedium erfolgt, insbesondere in der Gasphase (Eigen-Desinfektion), und wobei ein Erfassen und Abspeichern von den Desinfektionsvorgang kennzeichnenden Messwerten erfolgt, insbesondere der Dauer des thermischen Desinfizierens und/oder wenigstens einer Temperatur des Spülmediums oder der Komponenten (Eigendiagnose).

Das Aufbereitungs-Verfahren kann Teil des Desinfizierungs-Verfahrens sein. Das Desinfizierungs-Verfahren kann das Aufbereitungs-Verfahren umfassen, insbesondere chronologisch nach dem Schritt Desinfizieren.

Bevorzugt erfolgt das Desinfizieren auf rein thermische bzw. nicht-chemische Weise ohne Chemikalien, also frei von Chemikalien. Ein rein thermisches Desinfizieren von derartigen Vorrichtungen war bisher nicht möglich, insbesondere auch aufgrund von Vorschriften. Beispielsweise dürfen Hypothermiegeräte technisch gar nicht dafür geeignet sein, Temperaturen von über 40°C zu erzeugen. Ein rein thermisches Desinfizieren hat auch den Vorteil, dass kein Risiko von chemischen Rückständen besteht.

Das Risiko chemischer Rückstände ist durchaus vorhanden. Das Desinfizieren wird in vielen Fällen für eine Periode von ca. einem Monat verlangt. Anders ausgedrückt: Jeden Monat muss ein Desinfektionsvorgang durchgeführt werden.

Gemäß einer Ausführungsform umfass das Verfahren ferner auch den Schritt Entleeren eines Kaltbehälters der Desinfektionsvorrichtung; wobei beim thermischen Desinfizieren Spülmedium in der Gasphase vom Reservoir über einen offenen Zuleit-Bypass in den Kaltbehälter und wahlweise auch über einen offenen Ableit-Bypass weiter in einen Rücklaufbehälter der Desinfektionsvorrichtung geführt wird, insbesondere ohne Schaltung von Ventilen und durch einen im Reservoir erzeugten Dampfdruck. Dies ermöglicht ein robustes, zuverlässiges Verfahren, welches nicht zuletzt auch energie- und zeiteffizient durchgeführt werden kann.

Gemäß einer Ausführungsform umfasst das Verfahren zum Aufbereiten ferner die Schritte:
- Entleeren eines Reservoirs und/oder eines Rücklaufbehälters und/oder eines Kaltbehälters der Desinfektionsvorrichtung;
- zumindest teilweises Befüllen des Reservoirs und/oder Rücklaufbehälters und/oder Kaltbehälters mit frischem, ungebrauchtem Spülmedium bzw. Frischwasser;
- spülmediumautonomes thermisches Desinfizieren zumindest des Reservoirs und Rücklaufbehälters und wahlweise auch des Kaltbehälters oder weiteren Spülmedium führenden Komponenten der Desinfektionsvorrichtung, insbesondere bei einer Temperatur der Komponenten von mindestens 73°C;
- Erfassen wenigstens eines Messwertes und Regeln wenigstens einer Messgröße des Spülmediums, insbesondere der Temperatur des Spülmediums und/oder der Temperatur im bzw. von Reservoir, Rücklaufbehälter oder Kaltbehälter oder der Temperatur wenigstens einer der weiteren Komponenten;
- Aufzeichnen von Messwerten und Abspeichern erfasster Messwerte, insbesondere der Dauer des thermischen Desinfizierens und/oder wenigstens einer Temperatur des Spülmediums;
- Erstellen und Abspeichern eines Desinfektions-Protokolls bzw. Prüfberichts basierend auf den Messwerten. Der Prüfbericht kann durch Ausdruck oder Anzeige auf einer Anzeigeeinheit der Desinfektionsvorrichtung kenntlich gemacht werden.

Gemäß einer Ausführungsform werden das Reservoir und/oder der Rücklaufbehälter mit kaltem Spülmedium bzw. Spülmedium bei Raumtemperatur, insbesondere Kaltwasser befüllt oder durchgespült, insbesondere jeweils vordem Entleeren. Hierdurch kann die Desinfektionsvorrichtung abgekühlt werden, insbesondere auf zeitlich effiziente Weise.

Gemäß einer Ausführungsform wird der Desinfektionsvorrichtung ausschließlich Wasser in Trinkwasserqualität zugeführt oder ausschließlich elektrische Energie und Wasser in Trinkwasserqualität. Dies ermöglicht eine starke Autonomie. Ein Wasser- und/oder Stromanschluss kann ausreichen, so dass die Desinfektionsvorrichtung nahezu ortsunabhängig desinfizierbar ist.

Gemäß einer Ausführungsform wird das Desinfizieren bei einem Druck entsprechend Umgebungsdruck oder um bis zu 20mbar erhöhtem Umgebungsdruck durchgeführt.

Gemäß einer Ausführungsform wird das thermische Desinfizieren mittels Spülmedium in der Gasphase durchgeführt, indem vom Reservoir Spülmedium in der Gasphase in einen/den Kaltbehälter und in den Rücklaufbehälter geleitet wird, insbesondere bei offen in Reihe miteinander verbundenem Reservoir, Rücklaufbehälter und Kaltbehälter. Das Desinfizieren mittels Gasphase ermöglicht eine hohe Effizienz und Sicherheit. Dabei können alle Spülmedium führenden Behälter oder Armaturen der Desinfektionsvorrichtung in Reihe geschaltet werden. Hierdurch kann sichergestellt werden, dass all diese Komponenten gleichermaßen desinfiziert werden. Das Aufzeichnen der Temperatur des Spülmediums in der Gasphase stromab aller Komponenten ermöglicht eine belastbare Kontrolle des Aufbereitungsvorgangs.

Gemäß einer Ausführungsform wird das thermische Desinfizieren innerhalb einer Zeit von weniger als 120min., insbesondere weniger als 90min. durchgeführt, insbesondere zusatzfrei mittels Spülmedium in der Gasphase.

Gemäß einer Ausführungsform erfolgt beim Desinfizieren und/oder beim Aufbereiten auch ein Schritt einer Probenahme durch Einleiten von Spülmedium in einen Probenahme-Behälter der Desinfektionsvorrichtung. Hierdurch können Zuverlässigkeit und Kontrollmöglichkeiten oder Dokumentationsmöglichkeiten weiter verbessert werden.

### KURZE BESCHREIBUNG DER FIGUREN

In den nachfolgenden Figuren werden Ausführungsbeispiele näher erläutert. Es zeigen:
Fig. 1, 2 und 3 jeweils in einer Seitenansicht in schematischer Darstellung Bestandteile einer Desinfektionsvorrichtung gemäß einem Ausführungsbeispiel in unterschiedlichen Betriebszuständen;
Fig. 4 in einer Seitenansicht in schematischer Darstellung Bestandteile einer Desinfektionsvorrichtung gemäß einem weiteren Ausführungsbeispiel;
Fig. 5 in einer Seitenansicht in schematischer Darstellung Bestandteile einer Desinfektionsvorrichtung gemäß einem weiteren Ausführungsbeispiel;
Fig. 6 in schematischer Darstellung Schritte eines Verfahrens zum Desinfizieren ortsfester Leitungen gemäß einer Ausführungsform;
Fig. 7 in schematischer Darstellung Schritte eines Verfahrens zum Aufbereiten einer Desinfektionsvorrichtung gemäß einer Ausführungsform; und
Fig. 8 in schematischer Darstellung in Draufsicht einen exemplarischen Einsatzort für die Desinfektionsvorrichtung gemäß einer Ausführungsform.

### DETAILLIERTE BESCHREIBUNG DER FIGUREN

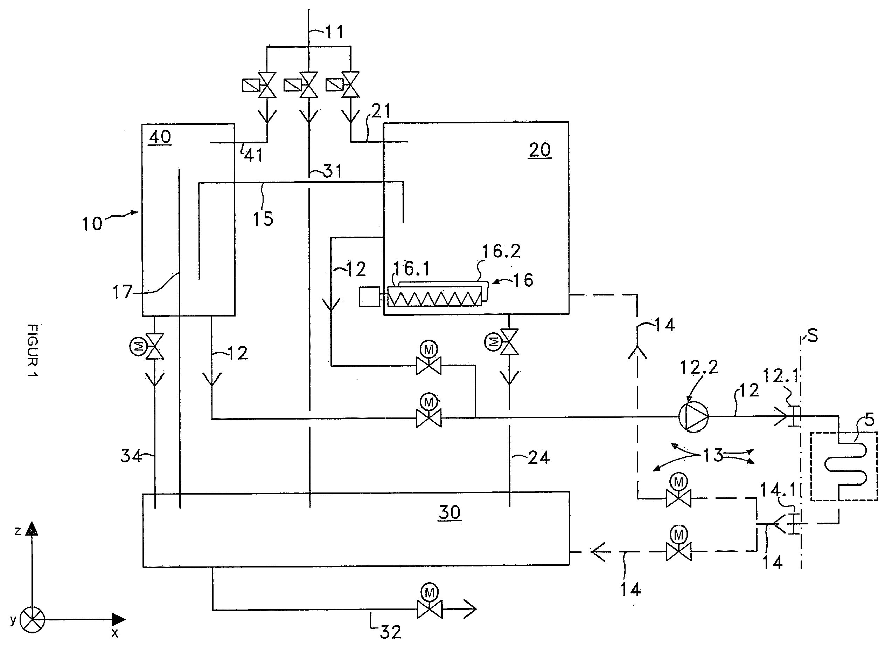

Bezüglich Bezugsziffern, die nicht explizit in einer der Figuren erläutert werden, wird auf die anderen Figuren verwiesen. Insbesondere werden die Figuren 1 bis 3 in Verbindung miteinander beschrieben.

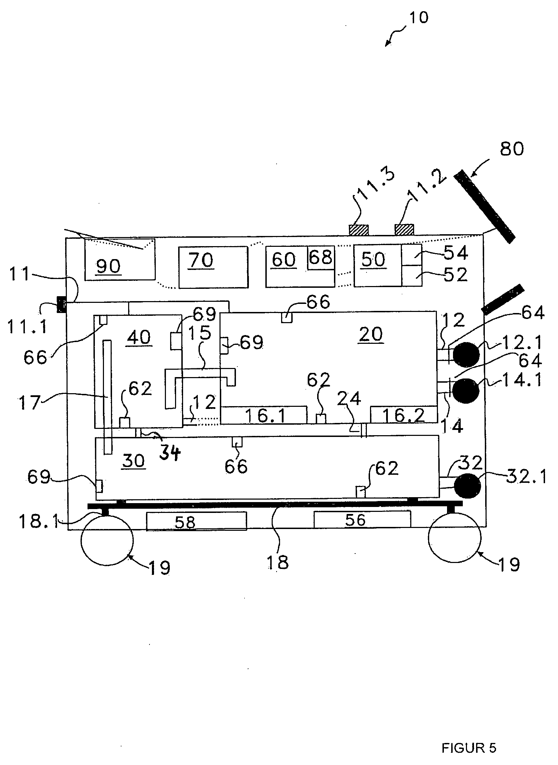

Fig. 1 zeigt eine Desinfektionsvorrichtung 10 mit einer Zuführleitung 11, einer Speiseleitung 12 mit einem Auslass 12.1, einer an der Speiseleitung 12 angeordneten Fördereinrichtung 12.2, einem Rücklauf 14 mit einem Einlass 14.1, einem Zuleit-Bypass 15 in Form eines beidseitig offenen Rohrs, einer Heizeinrichtung 16 mit einem ersten Heizelement 16.1 und einem zweiten Heizelement 16.2, einem Ableit-Bypass 17 in Form eines beidseitig offenen Rohrs, einem Reservoir 20 mit einer Zuleitung 21 und einer davon zu einem Rücklaufbehälter 30 führenden Leitung 24. Der Rücklaufbehälter 30 weist eine Zuleitung 31 und einen Ablauf 32 mit einem Auslass 32.1 auf (Fig. 5). Ferner ist ein Kaltbehälter 40 mit einer Zuleitung 41 vorgesehen, welcher über den Ableit-Bypass 17 sowie über eine Leitung 34 mit dem Rücklaufbehälter 30 verbunden ist.

Am Reservoir 20, Kaltbehälter 40 und Rücklaufbehälter 30 kann jeweils eine Zuleitung 21, 31,41 angeordnet sein. Die Desinfektionsvorrichtung 10 überbrückt am Auslass 12.1 und Einlass 14.1 jeweils eine Systemgrenze S und bildet mit einer ortsfesten Leitung 5 in verkuppeltem Zustand einen geschlossenen, hermetischen Umlauf 13, umfassend die zu desinfizierenden Leitungen.

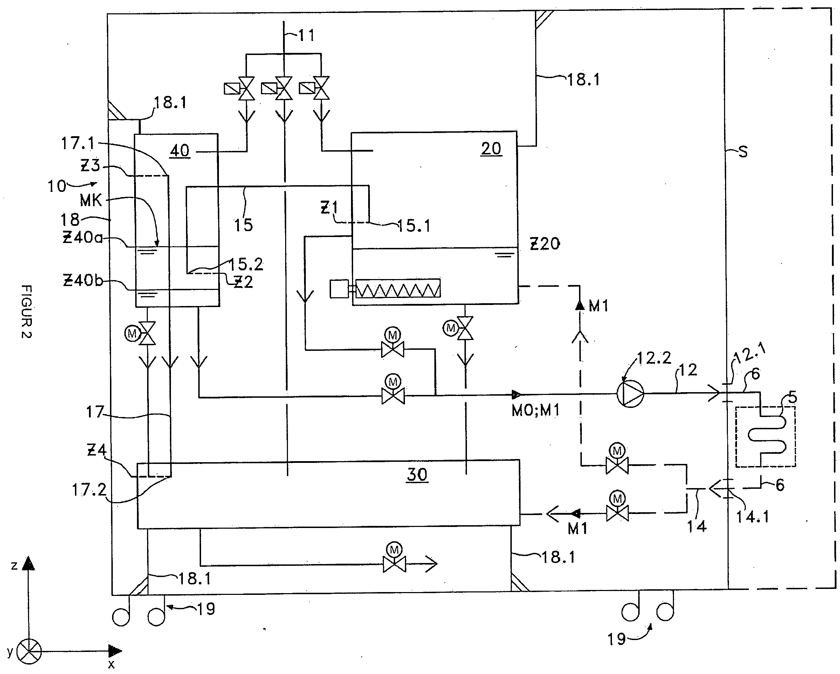

Fig. 2 zeigt in schematischer Art auch ein Gehäuse bzw. einen Rahmen 18, an welchem die einzelnen Behälter 20, 30, 40 mittels Stützen, Trägern oder Streben 18.1 abgestützt sein können. Der Rahmen kann dabei auch die Systemgrenze S bilden. Der Rahmen 18 oder einzelne Stützen 18.1 können direkt an Rollen 19 oder deren Achsen abgestützt sein. Die Desinfektionsvorrichtung 10 ist mobil, das heißt manuell oder motorisch verlagerbar.

In der Speiseleitung 12 kann ungebrauchtes (frisches) Spülmedium M0 oder auch gebrauchtes Spülmedium M1 geführt werden. Im Rücklauf 14 wird gebrauchtes Spülmedium M1 geführt, welches die ortsfeste Leitung 5 bereits durchströmt hat. Im Kaltbehälter 40 wird kaltes Spülmedium MK vorgehalten, insbesondere Wasser in Trinkwasserqualität. Die Fördereinrichtung 12.2 ist an der Speiseleitung 12 angeordnet, also auf der Druckseite des Umlaufs 13. Wahlweise kann die Fördereinrichtung auch auf einer Unterdruck-Seite bzw. Saugseite angeordnet sein. An Zu- und Ableitungen sowie an Leitungen zwischen den Behältern 20, 30, 40 sind Ventile M vorgesehen, insbesondere Motor- oder Magnetventile. Im Gegensatz dazu sind die beiden Bypässe 15, 17 beidseitig offen und ventillos.

Das erste freie Ende 15.1 im Reservoir 20 ist in einer Füllstands-Höhe z1 zumindest annähernd mittig in Bezug auf die Höhe des Reservoirs oder weiter oben im oberem Bereich des Reservoirs 20 oberhalb des maximalen Füllstandes angeordnet. Das zweite freie Ende 15.2 im Kaltbehälter 40 ist in einer Füllstands-Höhe z2 im unteren Drittel des Kaltbehälters angeordnet, also im unteren Beckenbereich des Kaltbehälters 40 unterhalb des Füllstandes (ausgehend vom Normalbetrieb bei Desinfektion der ortsfesten Leitung). Das erste freie Ende 17.1 im Kaltbehälter 40 ist in einer Füllstands-Höhe z3 im oberen Drittel des Kaltbehälters angeordnet, insbesondere derart, dass es auch eine Funktion als Überlauf für den Kaltbehälter 40 erfüllen kann. Das zweite freies Ende 17.2 im Rücklaufbehälter 30 ist in einer Füllstands-Höhe z4 im oberen Drittel des Rücklaufbehälters angeordnet.

Im Reservoir 20 liegt bei Eigen-Desinfektion ein Füllstand z20 vor, der unter der Füllstands-Höhe z1 liegt. Im Kaltbehälter 40 liegt ein Füllstand z40 bzw. z40a, z40b vor, der über oder unter der Füllstands-Höhe z2 liegen kann, je nach Betriebszustand. Beim Desinfizieren von ortsfesten Leitungen liegt im Kaltbehälter 40 ein Füllstand über der Höhe z2 vor, damit das zweite freie Ende 15.2 im Spülmedium bzw. Kaltwasser angeordnet ist und eine Art hydrostatische Sperre bildet (erster Füllstand z40a). Bei einer automatisierten Eigen-Desinfektion der Desinfektionsvorrichtung 10 kann der Kaltbehälter 40 ganz leer sein, oder aber der Füllstand wird zumindest unter der Höhe z2 gehalten (zweiter Füllstand z40b), so dass gasförmiges Spülmedium in den Kaltbehälter 40 gelangen kann.

In Fig. 2 ist ein Betriebszustand mit einem Füllstand z40a gezeigt, bei welchem Spülmedium vom Reservoir 20 nicht in den Kaltbehälter 40 gelangen kann, weder in der Gasphase noch in der Flüssigphase, zumindest nicht über den Zuleit-Bypass 15. Dessen zweites freies Ende 15.2 ist von Spülmedium bzw. Kaltwasser MK umgeben, so dass sich eine hydrostatische Sperre bildet.

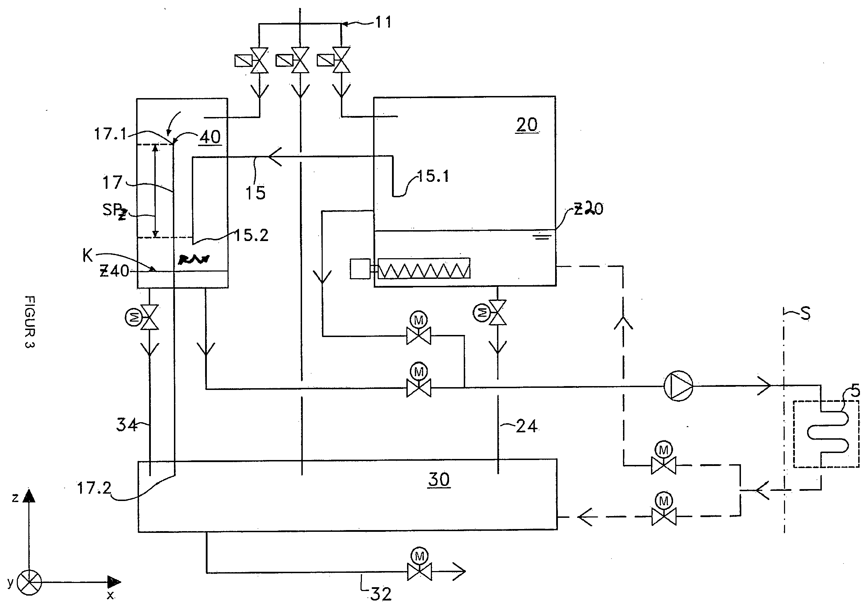

Fig. 3 zeigt einen Betriebszustand bei der Geräteaufbereitung. Die Desinfektionsvorrichtung 10 desinfiziert sich selbst, insbesondere vollautomatisch mit Dokumentation. Im Reservoir 20 liegt ein vergleichsweise kleines Volumen von Spülmedium in der Flüssigphase vor. Das Spülmedium umgibt die Heizelemente 16.1, 16.2, wird erhitzt, verdampft, durchläuft den offenen Zuleit-Bypass 15 und gelangt in den Kaltbehälter 40. Dort muss Spülmedium in der Gasphase vom zweiten freien Ende 15.2 zum ersten freien Ende 17.1 einen Strömungspfad SPz mit einer Länge entsprechend dem Abstand der beiden freien Enden zueinander zurücklegen. Die freien Enden 15.2, 17.1 können derart angeordnet sein, dass der vertikale Strömungspfad SPz möglichst lang ist, insbesondere mindestens 2/3 der Erstreckung des Kaltbehälters 40 in dieser Richtung. Dazu kann das zweite freie Ende 15.2 im unteren Drittel des Kaltbehälters 40 angeordnet sein, und das erste freie Ende 17.1 kann im oberen Drittel des Kaltbehälters angeordnet sein. Dies kann sicherstellen, dass Spülmedium in der Gasphase den Kaltbehälter vergleichsweise homogen erwärmt und zuverlässig desinfiziert. Ferner kann vom Ableit-Bypass 17 die Funktion eines Überlaufs für flüssiges Spülmedium in den Rückwasserbehälter erfüllt werden. Beim Erhitzen des Kaltbehälters 40 kann sich Kondensats K bilden, welches über die Leitung 34 dem Rücklaufbehälter 30 zugeführt werden kann. Die relative Position der freien Enden 15.2, 17.1 zueinander und in Bezug auf die Abmessungen des Kaltbehälters 40 ermöglicht also ein vergleichsweise homogenes Erwärmen und Bedampfen ohne das Erfordernis aufwändiger Steuerungs- oder Schaltungstechnik. Kondensat kann über den Ablauf 32 abgelassen werden. Die Leitungen 32, 24 und 34 können jeweils am tiefsten Punkt des entsprechenden Behälters 20, 30, 40 angeordnet sein. Die Behälter 20, 30, 40 können jeweils am tiefsten Punkt einen Auslass aufweisen, an welchen die jeweilige Leitung 32, 24, 34 angeschlossen ist. Dies ermöglicht das Entleeren des Systems ohne Pumparbeit.

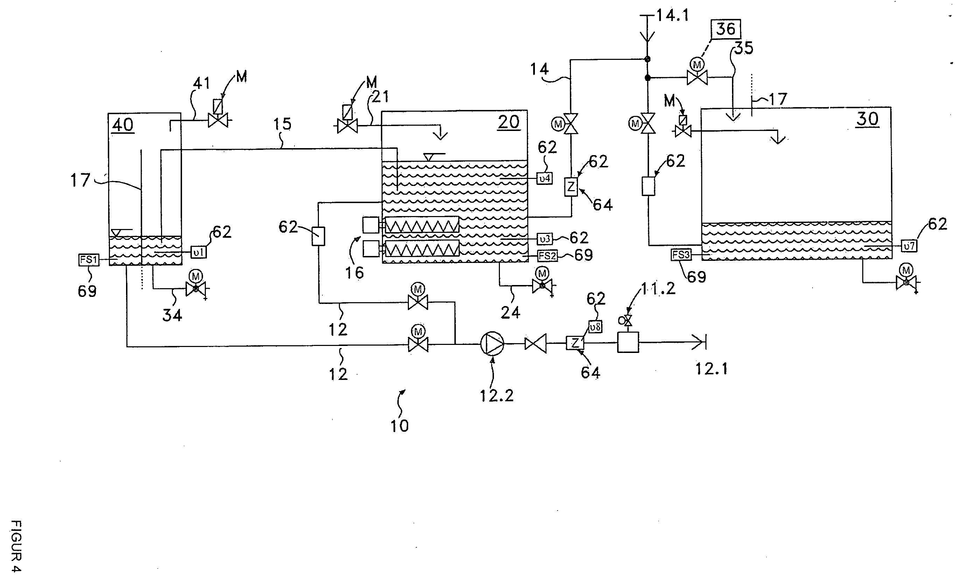

Fig. 4 zeigt eine Desinfektionsvorrichtung 10, welche auch eine Probenahmeleitung 35 umfasst, z.B. zur Entnahme von Kontroll-Proben für ein Labor. Die Probenahmeleitung 35 ist mit dem Rücklauf 14 und dem Reservoir 30 verbunden und kann mittels eines (Motor-)Ventils M geöffnet oder geschlossen werden. An das Ventil M kann wenigstens ein Probenahme-Behälter 36 gekuppelt sein. Eine Steuerungseinrichtung kann vorgeben, dass automatisch eine Probe genommen wird, insbesondere in Abhängigkeit einer Zeit bzw. in Abhängigkeit vom Verlauf eines Desinfektionsvorgangs. Hierzu kann die Steuerungseinrichtung das entsprechende Ventil M ansteuern, also z.B. eine Minute kurz vor dem Ende des Desinfektionsvorgangs öffnen, so dass gebrauchtes Spülmedium in die Probenahmeleitung 35 strömen kann. An der Probenahmeleitung 35 kann ferner ein Durchflusssensor angeordnet sein. Dann nämlich kann nach Durchfluss eines Mindestvolumens das Ventil M automatisch wieder geschlossen werden. Wahlweise kann das Ventil M standardmäßig für eine vordefinierte oder vorgebbare Zeitspanne geöffnet werden.

Die Fig. 4 zeigt eine Anordnung vergleichbar zu jener der Figuren 1 bis 3. In Fig. 4 ist der Rücklaufbehälter 30 der Übersichtlichkeit wegen rechts vom Reservoir 20 dargestellt. Zusätzlich ist eine zweckdienliche Position jeweils von Temperatursensoren 62 und Durchflusssensoren 64 (welche auch Temperatursensoren umfassen können) und Füllstandsensoren 69 gezeigt. Ferner ist ein Druckluftanschluss 11.2 an der Speiseleitung 12 angedeutet. An jedem Behälter 20, 30, 40 sind wenigstens ein Temperatursensor 62 und ein Füllstandsensor 69 angeordnet.

Fig. 5 zeigt weitere Komponenten einer Desinfektionsvorrichtung 10. Die Zuleitung 11 kann über einen Anschluss 11.1 für Spülmedium mit einer ortsfesten Versorgungsleitung oder einem Verbindungsschlauch (jeweils nicht dargestellt) verbunden werden. Ferner ist ein Druckluftanschluss 11.2 sowie ein Stromanschluss 11.3 vorgesehen.

Wie auch eine in den Figuren 1 bis 4 zuvor beschriebene Desinfektionsvorrichtung kann die in Fig. 5 gezeigte Desinfektionsvorrichtung 10 eine Steuerungseinrichtung 50, einen Speicher 52, insbesondere Programm- und/oder Datenspeicher, eine Recheneinheit 54, einen Energiespeicher 56, insbesondere Akkumulator, und einen Motor 58 bzw. eine Antriebseinheit aufweisen. Ferner kann eine Messeinrichtung 60 vorgesehen sein, welche z.B. eine Zeiterfassungseinheit 68 oder auch wenigstens einen der folgenden Sensoren aufweist oder zumindest daran gekoppelt ist: Temperatursensor 62, Durchflusssensor 64, Drucksensor 66, Füllstandsensor 69. Ferner kann eine Ausleseeinheit 70 zum Auslesen von Daten vorgesehen sein. An einer Anzeige- und Eingabeeinheit 80 können Bedieneingaben vorgenommen werden oder Daten angezeigt werden. Eine Ausgabeeinheit 90, insbesondere ein Drucker bzw. eine Druckereinheit ermöglicht, Prüfberichte zu dokumentieren, insbesondere in Papierform.

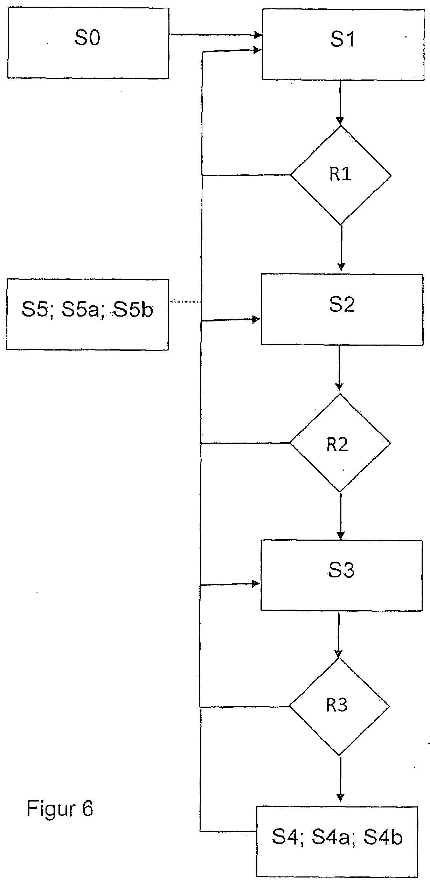

Fig. 6 zeigt Schritte eines Verfahrens zum automatisierten Desinfizieren von Innenflächen von ortsfesten Leitungen. In einem vorbereitenden Schritt S0 kann ein Bereitstellen einer verlagerbaren Desinfektionsvorrichtung erfolgen. In einem ersten Schritt S1 kann ein Anschließen eines Auslasses bzw. eines Einlasses erfolgen, insbesondere durch Verkuppeln mittels beidseitig abdichtenden Kupplungen. In einem zweiten Schritt S2 kann ein Einstellen oder Abrufen wenigstens eines Wertes oder Parameters erfolgen, insbesondere automatisch oder durch einen Bediener. Beispielsweise können ortsspezifische Angaben wie die Länge der ortsfesten Leitung oder deren Volumen eingegeben oder abgerufen werden. In einem dritten Schritt S3 kann ein Erfassen eines Messwertes bzw. ein Regeln einer Messgröße erfolgen, insbesondere durch mehrere Sensoren und die Steuerungseinrichtung. Insbesondere kann auch die Heizleistung basierend auf einem oder mehreren Temperaturmesswerten geregelt werden. Im Schritt S3 kann z.B. die Temperatur am Einlass erfasst werden. Es hat sich gezeigt, dass bereits eine Temperatur am Einlass im Bereich von 74°C bis 78°C, insbesondere 74°C bis 76°C, speziell 75°C ausreichen kann, um die gesamte jeweilige ortsfeste Leitung 5 zu desinfizieren. Hierzu kann auch der Durchfluss geregelt werden, beispielsweise im Bereich von 10-40L/min., insbesondere bei einer Leitung mit einem Durchmesser von z.B. 15-35mm.

Eine nicht allzu hohe Temperatur am Einlass bzw. ein vergleichsweise hoher Durchfluss liefert mehrere Vorteile. Zum einen ist der Energiebedarf vergleichsweise niedrig. Zum anderen werden die Armaturen vergleichsweise wenig beansprucht. Die Desinfektionsvorrichtung kann eingerichtet sein, im Umlauf ein Temperaturgefälle von z.B. max. 4 oder 5°C einzuhalten.

In einem vierten Schritt S4 kann ein spülmediumautonomes Desinfizieren der jeweiligen ortsfesten Leitung erfolgen, insbesondere auf rein thermische Weise ohne chemische Zusätze. Der Schritt S4 kann z.B. mittels einer Desinfektionstemperatur von bis zu 95°C durchgeführt werden. In einem Schritt S4a kann ein Durchspülen erfolgen, insbesondere unmittelbar vor und/oder nach dem Schritt des Desinfizierens. In einem Schritt S4b kann eine Probenahme erfolgen, insbesondere vollautomatisch. In einem fünften Schritt S5 kann ein Aufzeichnen und Abspeichern von Messwerten erfolgen. In einem Schritt S5a kann eine Messwertkontrolle erfolgen, insbesondere nach dem Schritt S4 oder S4a. In einem Schritt S5b kann ein Erstellen und Abspeichern eines Desinfektions-Protokolls bzw. Prüfberichts basierend auf erfassten Messwerten erfolgen.

Die einzelnen Schritte können unabhängig voneinander in den Verfahrensablauf integriert werden, insbesondere auch in einer Reihenfolge abweichend von der dargestellten Reihenfolge, z.B. auch synchron.

Dabei kann in einem ersten Regelungspunkt R1 ein Kontrollieren der Kupplung am Einlass oder Auslass erfolgen, insbesondere mittels Kontaktsensoren an einem jeweiligen Kupplungsteil. In einem zweiten Regelungspunkt R2 kann ein Kontrollieren einer Bedienereingabe oder eine Plausibilitätsprüfung erfolgen, insbesondere indem die Bedienereingabe mit hinterlegten Werten verglichen wird. Auf diese Weise können auch ortsspezifische, vollautomatisch aus einem ortsfest installierten Chip ausgelesene Daten verifiziert werden. In einem dritten Regelungspunkt R3 kann ein Regeln wenigstens einer Messgröße erfolgen, insbesondere zumindest auch der Zeit oder einer Temperatur. Eine Steuerungseinrichtung kann zum Ausführen der entsprechenden Regelung jeweils mit in diesem Zusammenhang vorgesehenen Sensoren gekoppelt sein. Die Regelungspunkte können unabhängig voneinander in den Verfahrensablauf integriert werden, insbesondere auch in einer Reihenfolge abweichend von der dargestellten Reihenfolge, z.B. auch synchron.

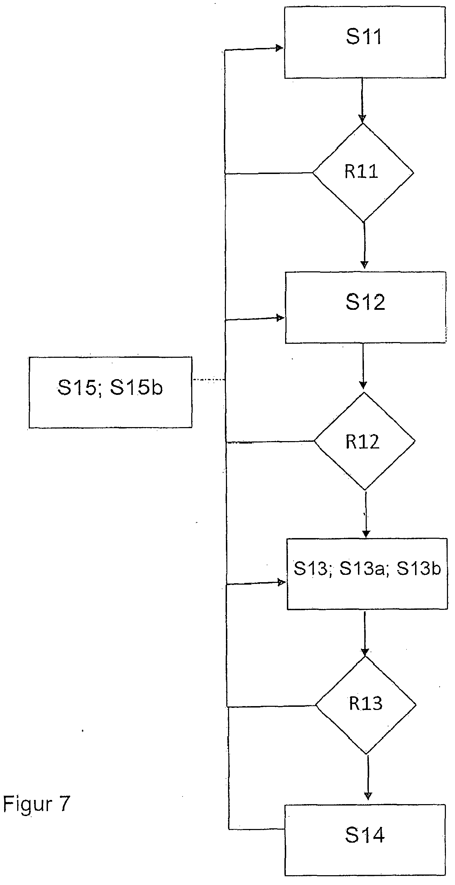

Fig. 7 zeigt Schritte eines Verfahrens zum Aufbereiten einer zuvor beschriebenen Desinfektionsvorrichtung eingerichtet zum Desinfizieren von ortsfesten Leitungen, insbesondere in Verbindung mit bzw. in Chronologie mit einem zuvor beschriebenen Verfahren zum automatisierten Desinfizieren von Innenflächen von ortsfesten Leitungen. In einem ersten Schritt S11 kann ein Entleeren des Reservoirs, eines Behälters und/oder einer Armatur der Desinfektionsvorrichtung erfolgen. Bevorzugt werden alle Behälter entleert. Hierdurch kann sichergestellt werden, dass die Behälter auch mittels Dampf bzw. Spülmedium in der Gasphase vollständig auf eine vergleichsweise hohe Temperatur gebracht werden können. In einem zweiten Schritt S12 kann ein zumindest teilweises Befüllen mit frischem, ungebrauchtem Spülmedium erfolgen, insbesondere ausschließlich des Reservoirs 20.

In einem dritten Schritt S13 kann thermisches Desinfizieren der Desinfektionsvorrichtung erfolgen, insbesondere mittels Spülmedium in der Gasphase. Dazu kann Spülmedium auf eine Soll-Temperatur von z.B. 102°C aufgeheizt werden, insbesondere im Reservoir. Es hat sich gezeigt, dass dabei schon das Verdampfen von ca. 2L Spülmedium für eine belastbare, zuverlässige Eigen-Desinfektion ausreichen kann. Hierdurch kann ein Dampfvolumen von ca. 3.500L erzeugt werden, entsprechend einem um z.B. den Faktor 10 größeren Volumen als das Innenvolumen der zu desinfizierenden Behälter bzw. Armaturen. Bevorzugt wird das Spülmedium in der Gasphase im Reservoir generiert, insbesondere mittels einer Heizeinrichtung umfassend wenigstens ein am oder im Reservoir angeordnetes Heizelement. Das Reservoir bzw. das Spülmedium kann dabei auf eine Temperatur über 100°C erhitzt werden, z.B. 102°C. Dies kann sicherstellen, dass die Gasphase erzeugt wird. Zwar ist der Übergangspunkt von der Flüssigphase in die Gasphase vom absoluten Druck abhängig. An einem Ort auf z.B. 2.500m Höhe tritt das Spülmedium daher schon deutlich unter 100°C in die Gasphase über. Daher kann die Soll-Temperatur für Spülmedium in der Gasphase im Reservoir einstellbar sein. Nichtsdestotrotz empfiehlt sich jedenfalls eine Temperatur von mindestens 102°C, insbesondere um dem Spülmedium ausreichend Energie zuzuführen um die gesamten Behälter und Armaturen auf über 80°C erhitzen zu können. Dies liefert eine hohe Sicherheit bezüglich der Effektivität des Desinfektionsvorgangs.

In einem Schritt S13a kann ein Befüllen oder Durchspülen mit kaltem Spülmedium erfolgen. In einem Schritt S13b kann eine Probenahme erfolgen, insbesondere vollautomatisch. In einem vierten Schritt S14 kann ein Erfassen eines Messwertes bzw. ein Regeln wenigstens einer Messgröße erfolgen, insbesondere zumindest auch der Zeit oder einer Temperatur. In einem fünften Schritt S15 kann ein Aufzeichnen und Abspeichern erfasster Messwerte erfolgen. In einem Schritt S15b kann ein Erstellen und Abspeichern eines Desinfektions-Protokolls bzw. Prüfberichts basierend auf erfassten Messwerten erfolgen.

Die einzelnen Schritte können unabhängig voneinander in den Verfahrensablauf integriert werden, insbesondere auch in einer Reihenfolge abweichend von der dargestellten Reihenfolge, z.B. auch synchron.

Dabei kann in einem ersten Regelungspunkt R11 ein Kontrollieren des Füllstandes erfolgen, insbesondere im Reservoir. In einem zweiten Regelungspunkt R12 kann ein Erfassen und Einstellen des Befüll-Volumens erfolgen, insbesondere indem ein Füllstand-Sensor ausgelesen wird, welcher einen für die Eigen-Desinfektion erforderlichen Mindest-Füllstand erfasst, z.B. 5 bis 10L. In einem dritten Regelungspunkt R13 kann ein Regeln wenigstens einer Messgröße erfolgen, insbesondere zumindest auch der Zeit oder einer Temperatur. Eine Steuerungseinrichtung kann zum Ausführen der entsprechenden Regelung jeweils mit in diesem Zusammenhang vorgesehenen Sensoren gekoppelt sein. Die Regelungspunkte können unabhängig voneinander in den Verfahrensablauf integriert werden, insbesondere auch in einer Reihenfolge abweichend von der dargestellten Reihenfolge, z.B. auch synchron.

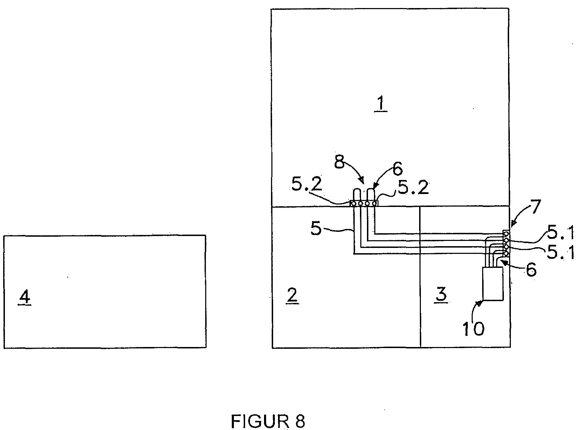

Die in der Fig. 7 und Fig. 8 beschriebenen Verfahrensschritte können miteinander kombiniert werden, insbesondere chronologisch hintereinander ausgeführt werden. Ein kombiniertes Verfahren kann wahlweise mit den Schritten S0, S1, ... oder mit den Schritten S11, S12, ... beginnen. Wahlweise kann auch eine Zeitprogrammierung erfolgen. Beispielsweise kann eine Tageszeit vorgegeben werden, zu welcher die Desinfektionsvorrichtung mit einer Eigen-Desinfektion starten soll. Dies kann die Verwendung der Desinfektionsvorrichtung in möglichst vollständig keimfreiem Zustand erleichtern. Insbesondere kann der Zeitpunkt für eine vollautomatische Eigen-Desinfektion derart vorgegeben werden, dass die Desinfektionsvorrichtung kurz vor einem Zeitraum wieder zur Verfügung steht, in welchem ortsfeste Leitungen desinfiziert werden können. In einer Klinik muss dies z.B. nachts zwischen 23:00Uhr und 05:00Uhr erfolgen.

Anhand der Fig. 8 ist ein Einsatzort für die Desinfektionsvorrichtung 10 in einer Klinik erläutert. Die Desinfektionsvorrichtung 10 ist in einem Vorraum 3 angeordnet, welcher an einen so genannten Einleitungs-Raum 2 (Einleitung) angrenzt, der zu einem Operationssaal 1 führt. Ferner ist ein von diesen Räumen separat angeordneter Bereich 4 für eine Geräteaufbereitung angedeutet, in welchem die Desinfektionsvorrichtung 10 abgestellt werden kann, insbesondere auch zwecks Eigen-Desinfektion. Zwischen dem Vorraum 3 und dem Operationssaal 1 verlaufen vier ortsfeste Leitungen 5, insbesondere in der Wand (unter dem Putz) oder im Bodenaufbau verlegt. Jede Leitung 5 weist ein erstes und zweites offenes Ende 5.1, 5.2 bzw. eine dort angeordnete Kupplung auf. Jede Leitung 5 stellt einen Eingang (IN) 7 und einen Ausgang (OUT) 8 bereit. Am Eingang 7 kann z.B. Trinkwasser zugeführt werden und am Ausgang 8 im Operationssaal 1 entnommen werden.

Der Eingang (IN) 7 übernimmt dabei die Funktion eines Einlasses der ortsfesten Leitung 5 während des Desinfektionsvorgangs. Die eigentliche Funktion des Einganges 7 kann hiervon unberührt bleiben. Gleiches gilt für den Ausgang (OUT) 8 der ortsfesten Leitung. Je nachdem, wie mehrere Leitungen 5 aneinander gekuppelt sind, können sich der Eingang (IN) und der Ausgang (OUT) verändern.

Die offenen Enden 5.1, 5.2 können jeweils paarweise mittels eines Verbindungsschlauchs 6 miteinander verbunden werden, so dass alle vier Leitungen 5 zusammen eine lange Leitung bilden. Dann sind im Operationssaal 1 zwei Verbindungsschläuche 6 jeweils an zwei Leitungen 5 angekuppelt, und im Vorraum 3 ist ein Verbindungsschlauch 6 an zwei Leitungen 5 angekuppelt. Vom Eingang 7 kann Spülmedium in Richtung Ausgang 8 strömen, dort im Verbindungsschlauch 6 umgelenkt werden und in eine weitere Leitung 5 geleitet werden, um schließlich an der Kupplungsstelle 5.2 wieder zurückgeführt zu werden.

Die Desinfektionsvorrichtung 10 kann zwei oder drei Schläuche umfassen bzw. bereithalten, mittels welchen die Desinfektionsvorrichtung 10 an zwei der Leitungen 5 gekuppelt werden kann. Diese Schläuche können den Verbindungsschläuchen 6 entsprechen oder auch als Teil einer Speiseleitung oder eines Rücklaufs ausgebildet sein.

Obwohl wenigstens ein beispielhaftes Ausführungsbeispiel in der vorhergehenden Beschreibung beschrieben wurde, können verschiedene Änderungen und Modifikationen vorgenommen werden. Das wenigstens eine beschriebene Ausführungsbeispiel ist lediglich exemplarisch beschrieben und nicht dazu vorgesehen, den Gültigkeitsbereich, die Anwendbarkeit oder die Konfiguration in irgendeiner Weise zu beschränken. Vielmehr stellt die vorhergehende Beschreibung dem Fachmann einen Plan oder eine technische Lehre zur Umsetzung wenigstens eines beispielhaften Ausführungsbeispiels zur Verfügung, wobei zahlreiche Änderungen in der Funktion und der Anordnung von beispielhaft beschriebenen Elementen oder Merkmalen gemacht werden können, ohne den Schutzbereich der aufgeführten Ansprüche oder rechtlicher Äquivalente zu verlassen.

## Patentansprüche

1. Desinfektionsvorrichtung (10) zum thermischen Desinfizieren von Innenflächen von ortsfesten Leitungen (5), insbesondere medizintechnischen Leitungen, mit:
einem Reservoir (20) für Spülmedium (M0, M1), zum Desinfizieren einer jeweiligen ortsfesten Leitung;
wenigstens einer vom Reservoir abgehenden Speiseleitung (12) mit einem Auslass (12.2) zum Kuppeln der Desinfektionsvorrichtung an eine jeweilige ortsfeste Leitung;
einer Heizeinrichtung (16) zum Bereitstellen des Spülmediums mit einer bezüglich Raumtemperatur erhöhten Soll-Temperatur, insbesondere mindestens 73°C;
einer mit der Heizeinrichtung gekoppelten Steuerungseinrichtung (50) zum Einstellen, Steuern oder Regeln einer Temperatur des Spülmediums;
wenigstens einer an die Steuerungseinrichtung gekoppelten Messeinrichtung (60) zum Erfassen wenigstens einer Messgröße des Spülmediums, insbesondere einer Temperatur;
einem Rücklauf (14) zum Zurückführen von Spülmedium aus der ortsfesten Leitung zurück in die Desinfektionsvorrichtung mit einem Einlass (14.1) zum Kuppeln der jeweiligen ortsfesten Leitung an den Rücklauf;
wobei die Desinfektionsvorrichtung spülmediumautonom ist.

2. Desinfektionsvorrichtung gemäß Anspruch 1, wobei die Desinfektionsvorrichtung eingerichtet ist, mittels der Speiseleitung und dem Rücklauf in Verbindung mit einer jeweiligen ortsfesten Leitung einen bezüglich der Desinfektionsvorrichtung auch extern angeordneten desinfizierbaren, hermetischen Umlauf (13) zu bilden.

3. Desinfektionsvorrichtung gemäß einem der vorhergehenden Ansprüche, wobei das Reservoir (20) ein Heißreservoir zum Bereitstellen und Vorhalten von Spülmedium mit einer Temperatur von mindestens 73°C, wobei die Desinfektionsvorrichtung zur rein thermischen Eigen-Desinfektion eingerichtet ist, insbesondere auch zur Eigendiagnose.

4. Desinfektionsvorrichtung gemäß einem der vorhergehenden Ansprüche, ferner mit einem mit dem Reservoir verbundenen Kaltbehälter (40), wobei der Kaltbehälter mittels eines Zuleit-Bypass (15), insbesondere beidseitig offenen Rohrs, mit dem Reservoir verbunden ist, welcher Zuleit-Bypass (15) zwei freie Enden (15.1, 15.2) aufweist, welche in den Kaltbehälter (40) und in das Reservoir (20) hineinragen, insbesondere bis zu einer vordefinierten Füllstands-Höhe (z1, z2).

5. Desinfektionsvorrichtung gemäß dem vorhergehenden Anspruch, wobei der Zuleit-Bypass (15) beidseitig offen und ventillos ohne Ventile ausgebildet ist, oder wobei sich der Zuleit-Bypass (15) U-förmig mit beidseitig nach unten offenen Enden (15.1, 15.2) erstreckt, oder wobei ein sich im Kaltbehälter (40) vertikal erstreckendes freies Ende (15.2) des Zuleit-Bypass ein sich im Reservoir (20) vertikal erstreckendes freies Ende (15.1) des Zuleit-Bypass in vertikaler Richtung überlappt, oder wobei sich das freie Ende (15.2) des Zuleit-Bypass im Kaltbehälter (40) weiter nach unten erstreckt als das freie Ende (15.1) des Zuleit-Bypass im Reservoir.

6. Desinfektionsvorrichtung gemäß einem der vorhergehenden Ansprüche, wobei die Desinfektionsvorrichtung (10) als mobiles Standgerät ausgebildet ist, wobei die Messeinrichtung (60) zum autonomen Erfassen von geografischen Daten eingerichtet ist, insbesondere mittels einer Ausleseeinheit (70).

7. Desinfektionsvorrichtung gemäß einem der vorhergehenden Ansprüche, wobei die Steuerungseinrichtung (50) zum Erstellen und Abspeichern eines Desinfektions-Protokolls basierend auf erfassten Messwerten eingerichtet ist, oder wobei die Desinfektionsvorrichtung eine Anzeige- oder Ausgabeeinheit (80, 90) zum Anzeigen oder Ausgeben eines/des Desinfektions-Protokolls aufweist.

8. Verwendung einer spülmediumautonomen Desinfektionsvorrichtung, insbesondere einer Desinfektionsvorrichtung (10) gemäß einem der vorhergehenden Ansprüche, zum thermischen Desinfizieren von Innenflächen von ortsfesten Leitungen (5).

9. Verwendung einer spülmediumautonomen Desinfektionsvorrichtung zum thermischen Desinfizieren von Innenflächen von medizintechnischen Leitungen (5) für Hypothermieanwendungen gegen Mykobakterien, insbesondere einer Desinfektionsvorrichtung (10) gemäß einem der vorhergehenden Vorrichtungsansprüche, in einem Vorraum (3) zu einem Operationssaal (1) zum Desinfizieren von in den Operationssaal führenden Leitungen (5), wobei Spülmedium (M0) ausschließlich einem Reservoir (20) der Desinfektionsvorrichtung entnommen wird und zur Desinfektionsvorrichtung zurückgeführt wird.

10. Verwendung wenigstens eines beidseitig offenen Bypass (15, 17) jeweils zwischen zwei Spülmedium aufnehmenden Behältern (20, 30, 40) einer Desinfektionsvorrichtung zum Desinfizieren von ortsfesten Leitungen (5), insbesondere zwei Bypass-Rohrleitungen zur Reihenschaltung von drei Behältern (20, 30, 40), zum Aufbereiten und Desinfizieren der Desinfektionsvorrichtung mittels Spülmedium (M0) in der Gasphase, insbesondere in einer Desinfektionsvorrichtung (10) gemäß einem der vorhergehenden Vorrichtungsansprüche.

11. Verfahren zum Desinfizieren von Innenflächen von ortsfesten Leitungen (5), insbesondere durch Verwendung einer Desinfektionsvorrichtung (10) gemäß einem der vorhergehenden Vorrichtungsansprüche, mit den Schritten:
- Einstellen wenigstens eines Wertes wenigstens einer Messgröße des Spülmediums oder wenigstens eines Verfahrensparameters oder Abrufen des wenigstens einen Wertes aus einem Speicher (52) der Desinfektionsvorrichtung;
- Erfassen wenigstens eines Messwertes und Regeln wenigstens einer Messgröße des Spülmediums (M0, M1);
- spülmediumautonomes Desinfizieren durch automatisiertes Einleiten von Spülmedium (M0) aus dem Reservoir in die ortsfeste Leitung (5) und Rückführen des eingeleiteten Spülmediums (M0) aus der ortsfesten Leitung zurück in die Desinfektionsvorrichtung;
- Aufzeichnen und Abspeichern von erfassten Messwerten des Spülmediums an einem Rücklauf (14) der Desinfektionsvorrichtung oder von der Dauer des Spülens.

12. Verfahren gemäß dem vorhergehenden Verfahrensanspruch, wobei das Desinfizieren auf rein thermische Weise erfolgt, insbesondere bei Soll-Temperaturen von mindestens 73°C, und wobei ein Erstellen und Abspeichern eines Desinfektions-Protokolls basierend auf erfassten Messwerten umfassend zumindest Temperaturwerte erfolgt.

13. Verfahren zum Aufbereiten einer Desinfektionsvorrichtung eingerichtet zum Desinfizieren von ortsfesten Leitungen (5), insbesondere einer Desinfektionsvorrichtung (10) gemäß einem der vorhergehenden Vorrichtungsansprüche, insbesondere in Verbindung mit einem Verfahren gemäß einem der vorhergehenden Verfahrensansprüche, wobei ein spülmediumautonomes rein thermisches Desinfizieren von Spülmedium führenden Komponenten (12, 13, 14, 15, 16, 17, 20, 30, 40) der Desinfektionsvorrichtung mittels in einem Reservoir (20) der Desinfektionsvorrichtung bereitgehaltenem Spülmedium (M0) erfolgt, insbesondere Spülmedium in der Gasphase, und wobei ein Erfassen und Abspeichern von den Desinfektionsvorgang kennzeichnenden Messwerten erfolgt.

14. Verfahren zum Aufbereiten gemäß dem vorhergehenden Verfahrensanspruch, mit dem Schritt
- Entleeren eines Kaltbehälters (40) der Desinfektionsvorrichtung; wobei beim thermischen Desinfizieren Spülmedium (M0) in der Gasphase vom Reservoir (20) über einen offenen Zuleit-Bypass (15) in den Kaltbehälter und wahlweise auch über einen offenen Ableit-Bypass (17) weiter in einen Rücklaufbehälter (30) der Desinfektionsvorrichtung geführt wird.

15. Verfahren zum Desinfizieren oder Verfahren zum Aufbereiten gemäß einem der vorhergehenden Verfahrensansprüche, ferner mit dem Schritt:
Probenahme durch Einleiten von Spülmedium in einen Probenahme-Behälter (36) der Desinfektionsvorrichtung (10).

## Geänderte Patentansprüche

### Geänderte Patentansprüche gemäss Regel 137(2) EPÜ.

**1.** Desinfektionsvorrichtung (10) zum thermischen Desinfizieren von Innenflächen von vorinstallierten ortsfesten medizintechnischen Haus-Leitungen (5) extern von der Desinfektionsvorrichtung, mit:
einem Reservoir (20) für Spülmedium (M0, M1), zum Desinfizieren einer jeweiligen ortsfesten Leitung;
wenigstens einer vom Reservoir abgehenden Speiseleitung (12) mit einem Auslass (12.2) zum Kuppeln der Desinfektionsvorrichtung an eine jeweilige ortsfeste Leitung;
einer Heizeinrichtung (16) zum Bereitstellen des Spülmediums mit einer bezüglich Raumtemperatur erhöhten Soll-Temperatur;
einer mit der Heizeinrichtung gekoppelten Steuerungseinrichtung (50) zum Einstellen, Steuern oder Regeln einer Temperatur des Spülmediums;
wenigstens einer an die Steuerungseinrichtung gekoppelten Messeinrichtung (60) zum Erfassen wenigstens einer Messgröße des Spülmediums umfassend eine Temperatur;
einem Rücklauf (14) zum Zurückführen von Spülmedium aus der ortsfesten Leitung zurück in die Desinfektionsvorrichtung mit einem Einlass (14.1) zum Kuppeln der jeweiligen ortsfesten Leitung an den Rücklauf;
wobei die Desinfektionsvorrichtung spülmediumautonom ohne Spülmediumzufuhr von extern ist.

**2.** Desinfektionsvorrichtung gemäß Anspruch 1, wobei die Desinfektionsvorrichtung eingerichtet ist, mittels der Speiseleitung und dem Rücklauf in Verbindung mit einer jeweiligen ortsfesten Leitung einen bezüglich der Desinfektionsvorrichtung auch extern angeordneten desinfizierbaren, hermetischen Umlauf (13) zu bilden.

**3.** Desinfektionsvorrichtung gemäß einem der vorhergehenden Ansprüche, wobei das Reservoir (20) ein Heißreservoir zum Bereitstellen und Vorhalten von Spülmedium mit einer Temperatur von mindestens 73°C ist, wobei die Desinfektionsvorrichtung zur rein thermischen Eigen-Desinfektion eingerichtet ist.

**4.** Desinfektionsvorrichtung gemäß einem der vorhergehenden Ansprüche, ferner mit einem mit dem Reservoir verbundenen Kaltbehälter (40), wobei der Kaltbehälter mittels eines Zuleit-Bypass (15) mit dem Reservoir verbunden ist, welcher Zuleit-Bypass (15) zwei freie Enden (15.1, 15.2) aufweist, welche in den Kaltbehälter (40) und in das Reservoir (20) hineinragen.

**5.** Desinfektionsvorrichtung gemäß dem vorhergehenden Anspruch, wobei der Zuleit-Bypass (15) beidseitig offen und ventillos ohne Ventile ausgebildet ist, und wobei ein sich im Kaltbehälter (40) vertikal erstreckendes freies Ende (15.2) des Zuleit-Bypass ein sich im Reservoir (20) vertikal erstreckendes freies Ende (15.1) des Zuleit-Bypass in vertikaler Richtung überlappt.

**6.** Desinfektionsvorrichtung gemäß einem der vorhergehenden Ansprüche, wobei die Desinfektionsvorrichtung (10) als mobiles Standgerät ausgebildet ist, wobei die Messeinrichtung (60) zum autonomen Erfassen von geografischen Daten eingerichtet ist.

**7.** Desinfektionsvorrichtung gemäß einem der vorhergehenden Ansprüche, wobei die Steuerungseinrichtung (50) zum Erstellen und Abspeichem eines Desinfektions-Protokolls basierend auf erfassten Messwerten eingerichtet ist, oder wobei die Desinfektionsvorrichtung eine Anzeige- oder Ausgabeeinheit (80, 90) zum Anzeigen oder Ausgeben eines/des Desinfektions-Protokolls aufweist.

**8.** Verwendung einer spülmediumautonomen Desinfektionsvorrichtung, insbesondere einer Desinfektionsvorrichtung (10) gemäß einem der vorhergehenden Ansprüche zum thermischen Desinfizieren von Innenflächen von vorinstallierten ortsfesten medizintechnischen Haus-Leitungen (5) extern von der Desinfektionsvorrichtung.

**9.** Verwendung einer spülmediumautonomen Desinfektionsvorrichtung zum thermischen Desinfizieren von Innenflächen von medizintechnischen Leitungen (5) für Hypothermieanwendungen gegen Mykobakterien, insbesondere einer Desinfektionsvorrichtung (10) gemäß einem der vorhergehenden Vorrichtungsansprüche, in einem Vorraum (3) zu einem Operationssaal (1) zum Desinfizieren von in den Operationssaal führenden vorinstallierten ortsfesten medizintechnischen Haus-Leitungen (5) extern von der Desinfektionsvorrichtung, wobei Spülmedium (M0) ausschließlich einem Reservoir (20) der Desinfektionsvorrichtung entnommen wird und zur Desinfektionsvorrichtung zurückgeführt wird.

**10.** Verwendung wenigstens eines beidseitig offenen Bypass (15, 17) jeweils zwischen zwei Spülmedium aufnehmenden Behältern (20, 30, 40) einer Desinfektionsvorrichtung zum Desinfizieren von vorinstallierten ortsfesten medizintechnischen Haus-Leitungen (5) extern von der Desinfektionsvorrichtung, zum Aufbereiten und Desinfizieren der Desinfektionsvorrichtung mittels Spülmedium (M0) in der Gasphase, insbesondere in einer Desinfektionsvorrichtung (10) gemäß einem der vorhergehenden Vorrichtungsansprüche.

**11.** Verfahren zum Desinfizieren von Innenflächen von vorinstallierten ortsfesten medizintechnischen Haus-Leitungen (5) extern von der Desinfektionsvorrichtung, insbesondere durch Verwendung einer Desinfektionsvorrichtung (10) gemäß einem der vorhergehenden Vorrichtungsansprüche, mit den Schritten:
- Einstellen wenigstens eines Wertes wenigstens einer Messgröße des Spülmediums oder wenigstens eines Verfahrensparameters oder Abrufen des wenigstens einen Wertes aus einem Speicher (52) der Desinfektionsvorrichtung;
- Erfassen wenigstens eines Messwertes und Regeln wenigstens einer Messgröße des Spülmediums (M0, M1) umfassend eine Temperatur;
- spülmediumautonomes Desinfizieren durch automatisiertes Einleiten von Spülmedium (M0) aus dem Reservoir in die ortsfeste Leitung (5) und Rückführen des eingeleiteten Spülmediums (M0) aus der ortsfesten Leitung zurück in die Desinfektionsvorrichtung;
- Aufzeichnen und Abspeichem von erfassten Messwerten des Spülmediums an einem Rücklauf (14) der Desinfektionsvorrichtung oder von der Dauer des Spülens.

**12.** Verfahren gemäß dem vorhergehenden Verfahrensanspruch, wobei das Desinfizieren auf rein thermische Weise erfolgt, und wobei ein Erstellen und Abspeichern eines Desinfektions-Protokolls basierend auf erfassten Messwerten umfassend zumindest Temperaturwerte erfolgt.

**13.** Verfahren zum Aufbereiten einer Desinfektionsvorrichtung eingerichtet zum Desinfizieren von vorinstallierten ortsfesten medizintechnischen Haus-Leitungen (5) extern von der Desinfektionsvorrichtung, insbesondere einer Desinfektionsvorrichtung (10) gemäß einem der vorhergehenden Vorrichtungsansprüche, insbesondere in Verbindung mit einem Verfahren gemäß einem der vorhergehenden Verfahrensansprüche, wobei ein spülmediumautonomes rein thermisches Desinfizieren von Spülmedium führenden Komponenten (12, 13, 14, 15, 16, 17, 20, 30, 40) der Desinfektionsvorrichtung mittels in einem Reservoir (20) der Desinfektionsvorrichtung bereitgehaltenem Spülmedium (M0) erfolgt, und wobei ein Erfassen und Abspeichem von den Desinfektionsvorgang kennzeichnenden Messwerten umfassend Temperaturmesswerte erfolgt.

**14.** Verfahren zum Aufbereiten gemäß dem vorhergehenden Verfahrensanspruch, mit dem Schritt
- Entleeren eines Kaltbehälters (40) der Desinfektionsvorrichtung; wobei beim thermischen Desinfizieren Spülmedium (M0) in der Gasphase vom Reservoir (20) über einen offenen Zuleit-Bypass (15) in den Kaltbehälter und wahlweise auch über einen offenen Ableit-Bypass (17) weiter in einen Rücklaufbehälter (30) der Desinfektionsvorrichtung geführt wird.

**15.** Verfahren zum Desinfizieren oder Verfahren zum Aufbereiten gemäß einem der vorhergehenden Verfahrensansprüche, ferner mit dem Schritt:
Probenahme durch Einleiten von Spülmedium in einen Probenahme-Behälter (36) der Desinfektionsvorrichtung (10).

**1.** Desinfektionsvorrichtung (10) zum thermischen Desinfizieren von Innenflächen von vorinstallierten ortsfesten medizintechnischen Haus-Leitungen (5) extern von der Desinfektionsvorrichtung, mit:
einem Reservoir (20) für Spülmedium (M0, M1), zum Desinfizieren einer jeweiligen ortsfesten Leitung;
wenigstens einer vom Reservoir abgehenden Speiseleitung (12) mit einem Auslass (12.2) zum Kuppeln der Desinfektionsvorrichtung an eine jeweilige ortsfeste Leitung;
einer Heizeinrichtung (16) zum Bereitstellen des Spülmediums mit einer bezüglich Raumtemperatur erhöhten Soll-Temperatur;
einer mit der Heizeinrichtung gekoppelten Steuerungseinrichtung (50) zum Einstellen, Steuern oder Regeln einer Temperatur des Spülmediums;
wenigstens einer an die Steuerungseinrichtung gekoppelten Messeinrichtung (60) zum Erfassen wenigstens einer Messgröße des Spülmediums umfassend eine Temperatur;
einem Rücklauf (14) zum Zurückführen von Spülmedium aus der ortsfesten Leitung zurück in die Desinfektionsvorrichtung mit einem Einlass (14.1) zum Kuppeln der jeweiligen ortsfesten Leitung an den Rücklauf;
wobei das Reservoir derart dimensioniert ist, dass die Desinfektionsvorrichtung beim Desinfizieren der ortsfesten Leitungen spülmediumautonom ohne Spülmediumzufuhr von extern ist.

**2.** Desinfektionsvorrichtung gemäß Anspruch 1, wobei die Desinfektionsvorrichtung eingerichtet ist, mittels der Speiseleitung und dem Rücklauf in Verbindung mit einer jeweiligen ortsfesten Leitung einen bezüglich der Desinfektionsvorrichtung auch extern angeordneten desinfizierbaren, hermetischen Umlauf (13) zu bilden.

**3.** Desinfektionsvorrichtung gemäß einem der vorhergehenden Ansprüche, wobei das Reservoir (20) ein Heißreservoir zum Bereitstellen und Vorhalten von Spülmedium mit einer Temperatur von mindestens 73°C ist, wobei die Desinfektionsvorrichtung zur rein thermischen Eigen-Desinfektion eingerichtet ist.

**4.** Desinfektionsvorrichtung gemäß einem der vorhergehenden Ansprüche, ferner mit einem mit dem Reservoir verbundenen Kaltbehälter (40), wobei der Kaltbehälter mittels eines Zuleit-Bypass (15) derart mit dem Reservoir verbunden ist, dass die Desinfektionsvorrichtung zur Eigen-Desinfektion mittels Spülmedium in der Gasphase eingerichtet ist, welcher Zuleit-Bypass (15) zwei freie Enden (15.1, 15.2) aufweist, welche in den Kaltbehälter (40) und in das Reservoir (20) hineinragen.

**5.** Desinfektionsvorrichtung gemäß dem vorhergehenden Anspruch, wobei der Zuleit-Bypass (15) beidseitig offen und ventillos ohne Ventile ausgebildet ist, und wobei ein sich im Kaltbehälter (40) vertikal erstreckendes freies Ende (15.2) des Zuleit-Bypass ein sich im Reservoir (20) vertikal erstreckendes freies Ende (15.1) des Zuleit-Bypass in vertikaler Richtung überlappt.

**6.** Desinfektionsvorrichtung gemäß einem der vorhergehenden Ansprüche, wobei die Desinfektionsvorrichtung (10) als mobiles Standgerät ausgebildet ist, wobei die Messeinrichtung (60) zum autonomen Erfassen von geografischen Daten eingerichtet ist.

**7.** Desinfektionsvorrichtung gemäß einem der vorhergehenden Ansprüche, wobei die Steuerungseinrichtung (50) zum Erstellen und Abspeichern eines Desinfektions-Protokolls basierend auf erfassten Messwerten eingerichtet ist, oder wobei die Desinfektionsvorrichtung eine Anzeige- oder Ausgabeeinheit (80, 90) zum Anzeigen oder Ausgeben eines/des Desinfektions-Protokolls aufweist.

**8.** Verwendung einer spülmediumautonomen Desinfektionsvorrichtung, insbesondere einer Desinfektionsvorrichtung (10) gemäß einem der vorhergehenden Ansprüche zum thermischen Desinfizieren von Innenflächen von vorinstallierten ortsfesten medizintechnischen Haus-Leitungen (5) extern von der Desinfektionsvorrichtung.

**9.** Verwendung einer spülmediumautonomen Desinfektionsvorrichtung zum thermischen Desinfizieren von Innenflächen von medizintechnischen Leitungen (5) für Hypothermieanwendungen gegen Mykobakterien, insbesondere einer Desinfektionsvorrichtung (10) gemäß einem der vorhergehenden Vorrichtungsansprüche, in einem Vorraum (3) zu einem Operationssaal (1) zum Desinfizieren von in den Operationssaal führenden vorinstallierten ortsfesten medizintechnischen Haus-Leitungen (5) extern von der Desinfektionsvorrichtung, wobei Spülmedium (M0) ausschließlich einem Reservoir (20) der Desinfektionsvorrichtung entnommen wird und zur Desinfektionsvorrichtung zurückgeführt wird.

**10.** Verwendung wenigstens eines beidseitig offenen Bypass (15, 17) jeweils zwischen zwei Spülmedium aufnehmenden Behältern (20, 30, 40) einer Desinfektionsvorrichtung zum Desinfizieren von vorinstallierten ortsfesten medizintechnischen Haus-Leitungen (5) extern von der Desinfektionsvorrichtung, zum Aufbereiten und Desinfizieren der Desinfektionsvorrichtung mittels Spülmedium (M0) in der Gasphase, insbesondere in einer Desinfektionsvorrichtung (10) gemäß einem der vorhergehenden Vorrichtungsansprüche.

**11.** Verfahren zum Desinfizieren von Innenflächen von vorinstallierten ortsfesten medizintechnischen Haus-Leitungen (5) extern von der Desinfektionsvorrichtung, insbesondere durch Verwendung einer Desinfektionsvorrichtung (10) gemäß einem der vorhergehenden Vorrichtungsansprüche, mit den Schritten:
- Einstellen wenigstens eines Wertes wenigstens einer Messgröße des Spülmediums oder wenigstens eines Verfahrensparameters oder Abrufen des wenigstens einen Wertes aus einem Speicher (52) der Desinfektionsvorrichtung;
- Erfassen wenigstens eines Messwertes und Regeln wenigstens einer Messgröße des Spülmediums (M0, M1) umfassend eine Temperatur;
- spülmediumautonomes Desinfizieren durch automatisiertes Einleiten von Spülmedium (M0) aus dem Reservoir in die ortsfeste Leitung (5) und Rückführen des eingeleiteten Spülmediums (M0) aus der ortsfesten Leitung zurück in die Desinfektionsvorrichtung;
- Aufzeichnen und Abspeichem von erfassten Messwerten des Spülmediums an einem Rücklauf (14) der Desinfektionsvorrichtung oder von der Dauer des Spülens.

**12.** Verfahren gemäß dem vorhergehenden Verfahrensanspruch, wobei das Desinfizieren auf rein thermische Weise erfolgt, und wobei ein Erstellen und Abspeichem eines Desinfektions-Protokolls basierend auf erfassten Messwerten umfassend zumindest Temperaturwerte erfolgt.

**13.** Verfahren zum Aufbereiten einer Desinfektionsvorrichtung eingerichtet zum Desinfizieren von vorinstallierten ortsfesten medizintechnischen Haus-Leitungen (5) extern von der Desinfektionsvorrichtung, insbesondere einer Desinfektionsvorrichtung (10) gemäß einem der vorhergehenden Vorrichtungsansprüche, insbesondere in Verbindung mit einem Verfahren gemäß einem der vorhergehenden Verfahrensansprüche, wobei ein spülmediumautonomes rein thermisches Desinfizieren von Spülmedium führenden Komponenten (12, 13, 14, 15, 16, 17, 20, 30, 40) der Desinfektionsvorrichtung mittels in einem Reservoir (20) der Desinfektionsvorrichtung bereitgehaltenem Spülmedium (M0) erfolgt, und wobei ein Erfassen und Abspeichern von den Desinfektionsvorgang kennzeichnenden Messwerten umfassend Temperaturmesswerte erfolgt, wobei beim thermischen Desinfizieren Spülmedium (M0) in der Gasphase vom Reservoir (20) über einen offenen Zuleit-Bypass (15) in den Kaltbehälter und wahlweise auch über einen offenen Ableit-Bypass (17) weiter in einen Rücklaufbehälter (30) der Desinfektionsvorrichtung geführt wird.

**14.** Verfahren zum Aufbereiten gemäß dem vorhergehenden Verfahrensanspruch, mit dem Schritt
- Entleeren eines Kaltbehälters (40) der Desinfektionsvorrichtung; wobei beim thermischen Desinfizieren Spülmedium (M0) in der Gasphase vom Reservoir (20) über einen offenen Zuleit-Bypass (15) in den Kaltbehälter und wahlweise auch über einen offenen Ableit-Bypass (17) weiter in einen Rücklaufbehälter (30) der Desinfektionsvorrichtung geführt wird.

**15.** Verfahren zum Desinfizieren oder Verfahren zum Aufbereiten gemäß einem der vorhergehenden Verfahrensansprüche, ferner mit dem Schritt:
Probenahme durch Einleiten von Spülmedium in einen Probenahme-Behälter (36) der Desinfektionsvorrichtung (10).

**1.** Desinfektionsvorrichtung (10) zum thermischen Desinfizieren von Innenflächen von vorinstallierten ortsfesten medizintechnischen Haus-Leitungen (5) extern von der Desinfektionsvorrichtung, mit:
einem Reservoir (20) für Spülmedium (M0, M1), zum Desinfizieren einer jeweiligen ortsfesten Leitung;
wenigstens einer vom Reservoir abgehenden Speiseleitung (12) mit einem Auslass (12.2) zum Kuppeln der Desinfektionsvorrichtung an eine jeweilige ortsfeste Leitung;
einer Heizeinrichtung (16) zum Bereitstellen des Spülmediums mit einer bezüglich Raumtemperatur erhöhten Soll-Temperatur, nämlich mindestens 73°C;
einer mit der Heizeinrichtung gekoppelten Steuerungseinrichtung (50) zum Einstellen, Steuern oder Regeln einer Temperatur des Spülmediums;
wenigstens einer an die Steuerungseinrichtung gekoppelten Messeinrichtung (60) zum Erfassen wenigstens einer Messgröße des Spülmediums umfassend eine Temperatur;
einem Rücklauf (14) zum Zurückführen von Spülmedium aus der ortsfesten Leitung zurück in die Desinfektionsvorrichtung mit einem Einlass (14.1) zum Kuppeln der jeweiligen ortsfesten Leitung an den Rücklauf;
wobei das Reservoir derart dimensioniert ist, dass die Desinfektionsvorrichtung beim Desinfizieren der ortsfesten Leitungen spülmediumautonom ohne Spülmediumzufuhr von extern ist.

**2.** Desinfektionsvorrichtung gemäß Anspruch 1, wobei die Desinfektionsvorrichtung eingerichtet ist, mittels der Speiseleitung und dem Rücklauf in Verbindung mit einer jeweiligen ortsfesten Leitung einen bezüglich der Desinfektionsvorrichtung auch extern angeordneten desinfizierbaren, hermetischen Umlauf (13) zu bilden.

**3.** Desinfektionsvorrichtung gemäß einem der vorhergehenden Ansprüche, wobei das Reservoir (20) ein Heißreservoir zum Bereitstellen und Vorhalten von Spülmedium mit einer Temperatur von mindestens 73°C ist, wobei die Desinfektionsvorrichtung zur rein thermischen Eigen-Desinfektion eingerichtet ist.

**4.** Desinfektionsvorrichtung gemäß einem der vorhergehenden Ansprüche, ferner mit einem mit dem Reservoir verbundenen Kaltbehälter (40), wobei der Kaltbehälter mittels eines Zuleit-Bypass (15) derart mit dem Reservoir verbunden ist, dass die Desinfektionsvorrichtung zur Eigen-Desinfektion mittels Spülmedium in der Gasphase eingerichtet ist, welcher Zuleit-Bypass (15) zwei freie Enden (15.1, 15.2) aufweist, welche in den Kaltbehälter (40) und in das Reservoir (20) hineinragen.

**5.** Desinfektionsvorrichtung gemäß dem vorhergehenden Anspruch, wobei der Zuleit-Bypass (15) beidseitig offen und ventillos ohne Ventile ausgebildet ist, und wobei ein sich im Kaltbehälter (40) vertikal erstreckendes freies Ende (15.2) des Zuleit-Bypass ein sich im Reservoir (20) vertikal erstreckendes freies Ende (15.1) des Zuleit-Bypass in vertikaler Richtung überlappt.

**6.** Desinfektionsvorrichtung gemäß einem der vorhergehenden Ansprüche, wobei die Desinfektionsvorrichtung (10) als mobiles Standgerät ausgebildet ist, wobei die Messeinrichtung (60) zum autonomen Erfassen von geografischen Daten eingerichtet ist.

**7.** Desinfektionsvorrichtung gemäß einem der vorhergehenden Ansprüche, wobei die Steuerungseinrichtung (50) zum Erstellen und Abspeichem eines Desinfektions-Protokolls basierend auf erfassten Messwerten eingerichtet ist, oder wobei die Desinfektionsvorrichtung eine Anzeige- oder Ausgabeeinheit (80, 90) zum Anzeigen oder Ausgeben eines/des Desinfektions-Protokolls aufweist.

**8.** Verwendung einer spülmediumautonomen Desinfektionsvorrichtunggemäß einem der vorhergehenden Ansprüche zum thermischen Desinfizieren von Innenflächen von vorinstallierten ortsfesten medizintechnischen Haus-Leitungen (5) extern von der Desinfektionsvorrichtung.

**9.** Verwendung einer spülmediumautonomen Desinfektionsvorrichtung gemäß einem der vorhergehenden Vorrichtungsansprüche zum thermischen Desinfizieren von Innenflächen von medizintechnischen Leitungen (5) für Hypothermieanwendungen gegen Mykobakterienin einem Vorraum (3) zu einem Operationssaal (1) zum Desinfizieren von in den Operationssaal führenden vorinstallierten ortsfesten medizintechnischen Haus-Leitungen (5) extern von der Desinfektionsvorrichtung, wobei Spülmedium (M0) ausschließlich einem Reservoir (20) der Desinfektionsvorrichtung entnommen wird und zur Desinfektionsvorrichtung zurückgeführt wird.

**10.** Verwendung wenigstens eines beidseitig offenen Bypass (15, 17) jeweils zwischen zwei Spülmedium aufnehmenden Behältern (20, 30, 40) einer Desinfektionsvorrichtung zum Desinfizieren von vorinstallierten ortsfesten medizintechnischen Haus-Leitungen (5) extern von der Desinfektionsvorrichtung, zum Aufbereiten und Desinfizieren der Desinfektionsvorrichtung mittels Spülmedium (M0) in der Gasphase, insbesondere in einer Desinfektionsvorrichtung (10) gemäß einem der vorhergehenden Vorrichtungsansprüche.

**11.** Verfahren zum Desinfizieren von Innenflächen von vorinstallierten ortsfesten medizintechnischen Haus-Leitungen (5) extern von der Desinfektionsvorrichtung, insbesondere durch Verwendung einer Desinfektionsvorrichtung (10) gemäß einem der vorhergehenden Vorrichtungsansprüche, mit den Schritten:
- Einstellen wenigstens eines Wertes wenigstens einer Messgröße des Spülmediums oder wenigstens eines Verfahrensparameters oder Abrufen des wenigstens einen Wertes aus einem Speicher (52) der Desinfektionsvorrichtung;
- Erfassen wenigstens eines Messwertes und Regeln wenigstens einer Messgröße des Spülmediums (M0, M1) umfassend eine Temperatur;
- spülmediumautonomes Desinfizieren durch automatisiertes Einleiten von Spülmedium (M0) aus dem Reservoir in die ortsfeste Leitung (5) und Rückführen des eingeleiteten Spülmediums (M0) aus der ortsfesten Leitung zurück in die Desinfektionsvorrichtung;
- Aufzeichnen und Abspeichem von erfassten Messwerten des Spülmediums an einem Rücklauf (14) der Desinfektionsvorrichtung oder von der Dauer des Spülens.

**12.** Verfahren gemäß dem vorhergehenden Verfahrensanspruch, wobei das Desinfizieren auf rein thermische Weise erfolgt, und wobei ein Erstellen und Abspeichem eines Desinfektions-Protokolls basierend auf erfassten Messwerten umfassend zumindest Temperaturwerte erfolgt.

**13.** Verfahren zum Aufbereiten einer Desinfektionsvorrichtung gemäß einem der vorhergehenden Vorrichtungsansprüche eingerichtet zum Desinfizieren von vorinstallierten ortsfesten medizintechnischen Haus-Leitungen (5) extern von der Desinfektionsvorrichtung, insbesondere in Verbindung mit einem Verfahren gemäß einem der vorhergehenden Verfahrensansprüche, wobei ein spülmediumautonomes rein thermisches Desinfizieren von Spülmedium führenden Komponenten (12, 13, 14, 15, 16, 17, 20, 30, 40) der Desinfektionsvorrichtung mittels in einem Reservoir (20) der Desinfektionsvorrichtung bereitgehaltenem Spülmedium (M0) erfolgt, und wobei ein Erfassen und Abspeichem von den Desinfektionsvorgang kennzeichnenden Messwerten umfassend Temperaturmesswerte erfolgt, wobei beim thermischen Desinfizieren Spülmedium (M0) in der Gasphase vom Reservoir (20) über einen offenen Zuleit-Bypass (15) in den Kaltbehälter und wahlweise auch über einen offenen Ableit-Bypass (17) weiter in einen Rücklaufbehälter (30) der Desinfektionsvorrichtung geführt wird.

**14.** Verfahren zum Aufbereiten gemäß dem vorhergehenden Verfahrensanspruch, mit dem Schritt
- Entleeren eines Kaltbehälters (40) der Desinfektionsvorrichtung; wobei beim thermischen Desinfizieren Spülmedium (M0) in der Gasphase vom Reservoir (20) über einen offenen Zuleit-Bypass (15) in den Kaltbehälter und wahlweise auch über einen offenen Ableit-Bypass (17) weiter in einen Rücklaufbehälter (30) der Desinfektionsvorrichtung geführt wird.

**15.** Verfahren zum Desinfizieren oder Verfahren zum Aufbereiten gemäß einem der vorhergehenden Verfahrensansprüche, wobei thermisches Desinfizieren von Innenflächen von medizintechnischen Leitungen (5) für Hypothermieanwendungen gegen Mykobakterien erfolgt, wobei Spülmedium (M0) ausschließlich einem Reservoir (20) der Desinfektionsvorrichtung entnommen wird und zur Desinfektionsvorrichtung zurückgeführt wird, ferner mit dem Schritt:
Probenahme durch Einleiten von Spülmedium in einen Probenahme-Behälter (36) der Desinfektionsvorrichtung (10).
